(19) Europäisches Patentamt
European Patent Office
Office européen des brevets

(11) Publication number: **0 239 306 B1**

(12) **EUROPEAN PATENT SPECIFICATION**

(45) Date of publication of patent specification: **02.06.93**

(51) Int. Cl.[5]: **C07D 209/86**, C07D 403/12, A61K 31/40, C07D 405/12

(21) Application number: **87302290.9**

(22) Date of filing: **18.03.87**

The file contains technical information submitted after the application was filed and not included in this specification

(54) **Tetrahydrocarbazole esters.**

(30) Priority: **27.03.86 CA 505362**

(43) Date of publication of application:
**30.09.87 Bulletin 87/40**

(45) Publication of the grant of the patent:
**02.06.93 Bulletin 93/22**

(84) Designated Contracting States:
**AT BE CH DE ES FR GB IT LI LU NL SE**

(56) References cited:
**EP-A- 0 200 391**
**EP-A- 0 234 708**
**GB-A- 1 511 169**
**US-A- 3 896 145**

**E. Schröder , "Pharmazeutische Chemie", (1982), Georg Thieme Verlag**

(73) Proprietor: **MERCK FROSST CANADA INC.**
**16711 Trans-Canada Highway**
**Kirkland Ouebec(CA)**

(72) Inventor: **Guidon, Yvan**
**7750 Jean Bourdon**
**Montreal Ouebec H4K 1H3(CA)**
Inventor: **Gillard, John W.**
**710 Westchester Baie d'Urf E.**
**Montreal Ouebec H9X 2S1(CA)**
Inventor: **Yoakim, Christiane**
**5770 Cote St. Luc Road Apt. 12**
**Montreal Ouebec H3X 2C1(CA)**

(74) Representative: **Cole, William Gwyn**
**European Patent Department, Merck & Co., Inc., Terlings Park, Eastwick Road**
**Harlow, Essex CM20 2OR (GB)**

**EP 0 239 306 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

This invention relates to prostaglandin antagonists useful in treating a variety of conditions, such as allergic asthma where excessive contractile activity of prostaglandins and prostaglandin biosynthetic intermediates occur.

These compounds antagonize the actions of contractile prostaglandins, such as $PGF_{2\alpha}$, $PGG_2$, $PGH_2$, $PGD_2$ and $TXA_2$. The use of agents which act as prostaglandin antagonists offers new approaches to therapy in a number of disease states. For example, certain prostaglandins, such as $PGF_{2\alpha}$, $PGD_2$, $PGG_2$, and $PGH_2$, are potent bronchospastic agents. Indeed human asthmatics have been shown to be especially sensitive to the bronchial constricting action of $PGF_{2\alpha}$.

The compounds of the present invention are also antithrombotic agents. Thus, they are useful in the treatment and/or prevention of thromboembolic diseases such as arterial thrombosis and those involving platelet deposition, e.g. prothesis.

In addition to the involvement of contractile prostaglandins in asthma, prostaglandins are known to play a role in other allergic conditions, as well as, diarrhea, hypertension, angina, platelet aggregation, cerebral spasm, cerebral ischemia, arrythmia, circulatory shock, sudden death, atherosclerosis, myocardial ischemia, premature labor, spontaneous abortion, dysmenorrhea, glomerular nephritis, and systemic lupus erythematosis. Consequently, the compounds of this invention will alleviate the above mentioned diseases.

In addition to the prostaglandin antagonist actions, the compounds of this invention are inhibitors of the biosynthesis of 5-lipoxygenase metabolites of arachidonic acid, such as 5-HPETE, 5-HETE and the leukotrienes. Leukotrienes $B_4$, $C_4$, $D_4$ and $E_4$ are known to contribute to various disease conditions such as asthma, psoriasis, pain, ulcers and systemic anaphylaxis. Thus inhibition of the synthesis of such compounds will alleviate these and other leukotriene-related disease states.

The compounds of the present invention may be used to treat or prevent mammalian (especially, human) disease states such as erosive gastritis; erosive esophagitis; ethanol-induced hemorrhagic erosions; hepatic ischemia; noxious agent induced damage or necrosis of hepatic, pancreatic, renal, or myocardial tissue; liver parenchymal damage caused by hepatoxic agents such as $CCl_4$ and D-galactosamine; ischemic renal failure; disease-induced hepatic damage; bile salt induced pancreatic or gastric damage; trauma- or stress-induced cell damage; and glycerol-induced renal failure.

Certain 9-benzyl-1,2,3,4-tetrahydrocarbazole acetic acids or esters thereof are shown as chemical intermediates in the preparation of carbazoles that are known in the art as antiinflammatory, analgesic and anti-rheumatic agents (see U.S. Patent 3,896,145 and British Patent 1,385,620). Certain 9-benzyl-1,2,3,4-tetrahydrocarbazole carboxylic acids are known in the art as anti-inflammatory, analgesic and anti-rheumatic agents (see U.S. Patents 3,868,387; 4,009,181; 3,905,998 and 3,758,496), and 9-benzylcarbazole carboxylic acids (U.S. Patents 3,956,295 and 4,057,640) and 9-benzylcarbazole acetic acids and esters thereof (U.S. Patent 3,896,145 and British Patent 1,385,620) are known as anti-inflammatory, analgesic and anti-rheumatic agents. None of these compounds, however, are shown to be prostaglandin or thromboxane antagonists or inhibitors of leukotriene biosynthesis.

British Patent 1,511,169 describes inter alia a family of substituted 1,2,3,4-tetrahydrocarbazole-1-acetic acid derivatives. These compounds are alleged to exhibit anti-inflammatory activity at relatively low dose levels.

### DESCRIPTION OF THE INVENTION

One embodiment of the present invention is a pharmaceutical composition containing a compound of Formula I:

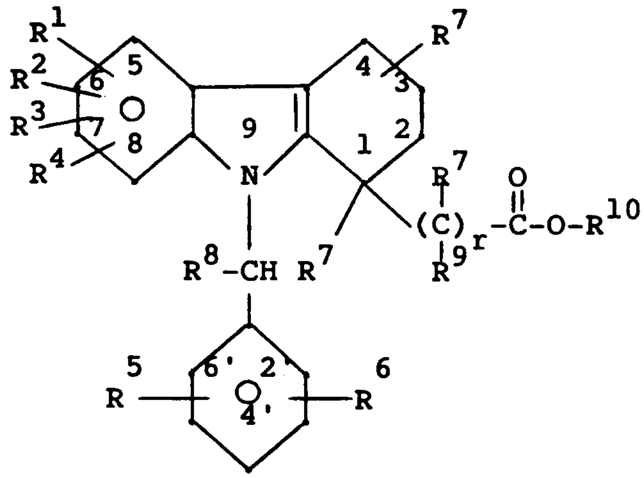

I

wherein:

$R^1$, $R^2$, $R^3$, $R^4$, $R^5$ and $R^6$ are each independently selected from:

(1) hydrogen;

(2) alkyl having 1 to 6 carbons;

(3) alkenyl having 2 to 6 carbons;

(4) $-(CH_2)_nM$

wherein n is 0 to 3 and M is

a) $OR^{11}$;

b) halogen;

c) $CF_3$;

d) $SR^{11}$;

e) phenyl or substituted phenyl;

f) $COOR^{12}$;

g)

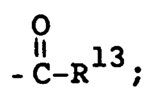

h) tetrazole;

i)

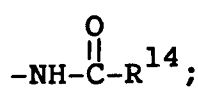

j) $-NR^{12}R^{12}$;

k) $-NHSO_2R^{15}$;

l)

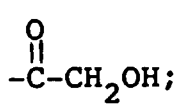

m) $-SOR^{11}$;

n) $-CONR^{12}R^{12}$;

o) $-SO_2NR^{12}R^{12}$;

p) $-SO_2R^{11}$;

q) $NO_2$;

r)

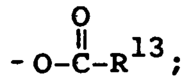

s)

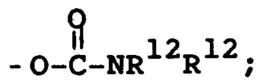

t)

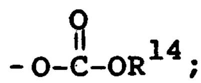

u) CN;

v) $N_3$;

$R^7$ is H or alkyl of 1 to 6 carbons;

$R^8$ is H or alkyl of 1 to 6 carbons;

each $R^9$ is independently H, OH, $C_1$ to $C_4$-O-alkyl or alkyl of 1 to 4 carbons;

$R^{10}$ is lower alkyl, substituted or unsubstituted 2-phenethyl, substituted or unsubstituted benzyl, substituted or unsubstituted phenyl, or

$-(CH_2)_t-C(R^7)_2-(CH_2)_t-R^{16}$

each $R^{11}$ is independently H; $C_1$ to $C_6$ alkyl; benzyl; phenyl or substituted phenyl;

each $R^{12}$ is independently H, phenyl, benzyl or $C_1$ to $C_6$ alkyl;

each $R^{13}$ is independently H, $(CH_2)_mCOOR^{12}$ wherein m is 0 to 4, $C_1$ to $C_6$ alkyl, $CF_3$, phenyl, or substituted phenyl;

each $R^{14}$ is independently $C_1$ to $C_6$ alkyl, benzyl or phenyl;

each $R^{15}$ is independently $C_1$ to $C_6$ alkyl, 4-methylphenyl, phenyl, or $CF_3$;

$R^{16}$ is A) a monocyclic or bicyclic heterocyclic radical containing from 3 to 12 nuclear carbon atoms and 1 or 2 nuclear heteroatoms selected from N, S, or O and with each ring in the heterocyclic radical being formed of 5 or 6 atoms, or B) the radical $W-R^{17}$;

$R^{17}$ contains up to 21 carbon atoms and is (1) a hydrocarbon radical or (2) an acyl radical of an organic acyclic or monocyclic carboxylic acid containing not more than 1 heteroatom in the ring;

W is O, S or NH;

r is 1 to 6

t is 0 to 3

or a pharmaceutically acceptable salt thereof.

Preferred compositions of the present invention contain a compound of formula I wherein:

$R^1$, $R^2$, $R^3$, $R^4$, $R^5$ and $R^6$ are each independently selected from:

(1) hydrogen;

(2) alkyl having 1 to 6 carbons;

(3) alkenyl having 2 to 6 carbons;

(4) $-(CH_2)_nM$

wherein n is 0 or 1 and M is as defined previously for Formula I;

and the remaining substituents are as defined previously for Formula I.

More preferred compositions of the present invention contain a compound of Formula I. wherein:

$R^1$, $R^2$, $R^3$, $R^4$, $R^5$ and $R^6$ are each independently selected from:

(1) hydrogen;

(2) alkyl having 1 to 6 carbons;

(3) alkenyl having 2 to 6 carbons;

(4) M, wherein M is as defined initially for Formula I;

r is 1 or 2; and the remaining substituents are as defined initially for Formula I.

Most preferred compositions of the present invention contain a compound of Formula I. wherein:

$R^1$, $R^2$, $R^3$, $R^4$, $R^5$ and $R^6$ are each independently selected from:

(1) hydrogen;

(2) alkyl having 1 to 6 carbons;

(3) M wherein M is

a) $OR^{11}$;

b) halogen;

c) $CF_3$;

d) $SR^{11}$;

e) $COOR^{12}$;

f)

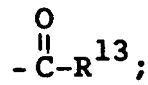

g) tetrazole;

h) $-SOR^{11}$;

i) $-CONR^{12}R^{12}$;

j) $-SO_2NR^{12}R^{12}$;

k) $-SO_2R^{11}$;

l)

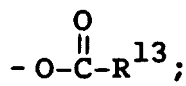

m) CN;

n) $N_3$;

each $R^9$ is independently H, or alkyl of 1 to 4 carbons;

$R^{10}$ is lower alkyl, $-C(R^7)_2-O-C(O)-R^7$

or

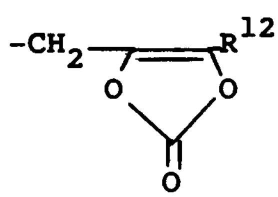

r is 1 and the remaining substituents are as defined initially for Formula I.

Another embodiment of the present invention relates to novel compounds of Formula I:

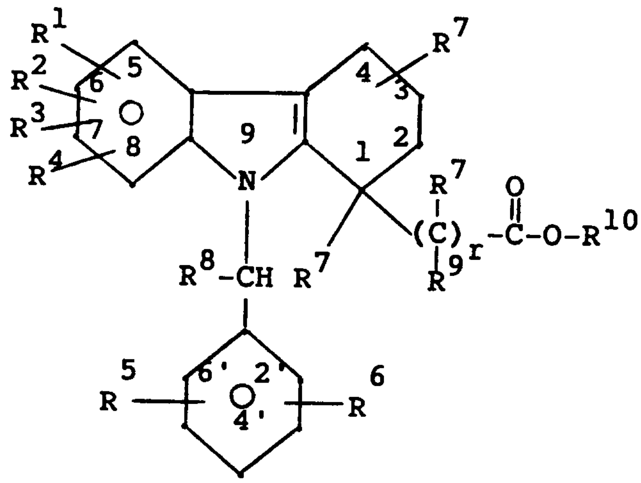

I

wherein:

$R^1$, $R^2$, $R^3$, $R^4$, $R^5$ and $R^6$ are each independently selected from:

(1) hydrogen;

(2) alkyl having 1 to 6 carbons;

(3) alkenyl having 2 to 6 carbons;

(4) $-(CH_2)_nM$

wherein n is 0 to 3 and M is

a) $OR^{11}$;

b) halogen;

c) $CF_3$;

d) $SR^{11}$;

e) phenyl or substituted phenyl;

f) $COOR^{12}$;

g)

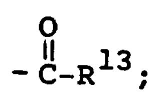

h) tetrazole;

i)

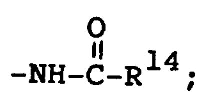

j) $-NR^{12}R^{12}$;

k) $-NHSO_2R^{15}$;

l)

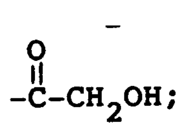

m) $-SOR^{11}$;

n) $-CONR^{12}R^{12}$;
o) $-SO_2NR^{12}R^{12}$;
p) $-SO_2R^{11}$;
q) $NO_2$;
r)

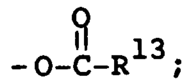

s)

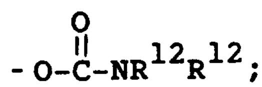

t)

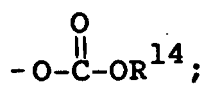

u) CN;
v) $N_3$;

provided that when $R^1$, $R^2$, $R^3$ or $R^4$ is alkyl having 1 to 6 carbons, it is not located at position 6;

$R^7$ is H or alkyl of 1 to 6 carbons;

$R^8$ is H or alkyl of 1 to 6 carbons;

each $R^9$ is independently H, OH, $C_1$ to $C_4$-O-alkyl or alkyl of 1 to 4 carbons;

$R^{10}$ is lower alkyl, substituted or unsubstituted 2-phenethyl, substituted or unsubstituted benzyl, substituted or unsubstituted phenyl, or

$-(CH_2)_t-C(R^7)_2-(CH_2)_t-R^{16}$

each $R^{11}$ independently is H; $C_1$ to $C_6$ alkyl; benzyl; phenyl or substituted phenyl;

each $R^{12}$ is independently H, phenyl, benzyl or $C_1$ to $C_6$ alkyl; and,

each $R^{13}$ is independently H, $(CH_2)_mCOOR^{12}$ wherein m is 0 to 4, $C_1$ to $C_6$ alkyl, $CF_3$, phenyl, or substituted phenyl;

each $R^{14}$ is $C_1$ to $C_6$ alkyl, benzyl or phenyl;

each $R^{15}$ is $C_1$ to $C_6$ alkyl, 4-methylphenyl, phenyl, or $CF_3$;

$R^{16}$ is A) a monocyclic or bicyclic heterocyclic radical containing from 3 to 12 nuclear carbon atoms and 1 or 2 nuclear heteroatoms selected from N, S, or O and with each ring in the heterocyclic radical being formed of 5 or 6 atoms, or B) the radical $W-R^{17}$;

$R^{17}$ contains up to 21 carbon atoms and is (1) a hydrocarbon radical or (2) an acyl radical of an organic acyclic or monocyclic carboxylic acid containing not more than 1 heteroatom in the ring;

W is O, S or NH;

r is 1 to 6

t is 0 to 3

or a pharmaceutically acceptable salt thereof.

As used herein, the terms "each independently" or the equivalents thereof are employed to describe a number of possible position isomers and/or structural variations. For example, as described above, the following unit is attached to position 1 of the tetrahydrocarbazole ring:

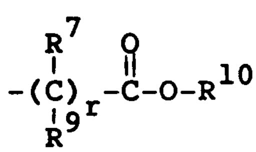

The letter r represents possible alkane chains of from 1 to 6 carbon atoms, each having the $R^7$ and $R^9$ substituent groups. On each carbon atom of the alkane chain, the $R^7$ and/or $R^9$ substituent may be different. The above description therefore contemplates structures such as the following for the segment $-(CR^7R^9)_r-$:

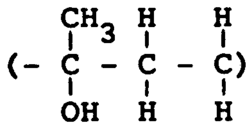,

$(-CH_2-)$

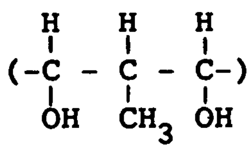, 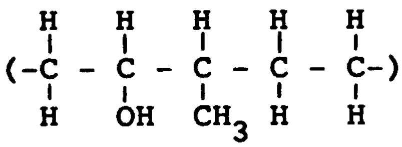,

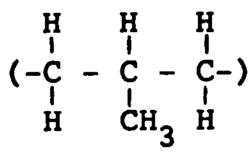,

and the like.

Substituted phenyl, substituted benzyl, and substituted phenethyl signifies the presence of 1 or 2 substituents on the benzene ring selected from $C_1$ to $C_3$ alkyl, halogen, CN, $CF_3$, $COOR^{12}$, $CH_2COOR^{12}$, or $C_1$ to $C_3$ alkoxy.

The alkyl groups referred to above may be straight chain or branched or may include cycloalkyl groups. As used herein, the term "lower" as applied to alkyl, acyl, alkoxy and the like, unless stated otherwise refers to groups having 1 to 6 carbon atoms. Halogen or halo means fluoro, chloro, bromo and/or iodo.

The esters when $R^{10}$ is $-(CH_2)_t-C(R^7)_2-(CH_2)_t-R^{16}$ are intended to include the esters such as are described by Saari, et al, J. Med. Chem., 21, 746-753 (1978) and Sakamoto, et al, Chem. Pharm. Bull., 32, 2241-2248 (1984) which are hereby incorporated by reference.

Pharmaceutically acceptable salts of the compounds described herein are included within the scope of the present invention. Such salts may be prepared from pharmaceutically acceptable non-toxic bases including inorganic bases and organic bases. Salts derived from inorganic bases include sodium, potassium, lithium, ammonium, calcium, magnesium, ferrous, zinc, copper, manganous, aluminum, ferric, manganic salts and the like. Particularly preferred are the potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, tri-methylamine, diethanolamine, diethylamine, triethylamine, tripropylamine, ethanolamine, 2-dimethylaminoethanol, 2-diethylamino -ethanol, tomethamine, lysine, arginine, histidine, caffeine, procaine, hydrabamine, choline, imidazole, betaine, ethylenediamine, glucosamine, methylglucamine, theobromine, purines piperazine, N,N-dibenzyl- ethylenediamine, piperidine, N-ethyl-piperidine, morpholine, N-ethylmorpholine, polyamine resins and the like.

Preferred novel compounds of the present invention comprise the compound of Formula I wherein:

$R^1$, $R^2$, $R^3$, $R^4$, $R^5$ and $R^6$ are each independently selected from:

(1) hydrogen;

(2) alkyl having 1 to 6 carbons;

(3) alkenyl having 2 to 6 carbons;

(4) $-(CH_2)_nM$

wherein n is 0 or 1 and M is as defined previously for the compounds of Formula I;

and the remaining substituents are as defined previously for the compounds of Formula I.

More preferred novel compounds of the present invention comprise a compound of Formula I wherein:

$R^1$, $R^2$, $R^3$, $R^4$, $R^5$ and $R^6$ are each independently selected from:

(1) hydrogen;
(2) alkyl having 1 to 6 carbons;
(3) alkenyl having 2 to 6 carbons;
(4) M, wherein M is as defined initially for the compounds of Formula I;

r is 1 or 2; and the remaining substituents are as defined initially for the compounds of Formula I.

Most preferred novel compounds of the present invention comprise the compound of Formula I wherein:

$R^1$, $R^2$, $R^3$, $R^4$, and $R^5$ are each independently selected from:

(1) hydrogen;
(2) alkyl having 1 to 6 carbons;
(3) M wherein M is
  a) $OR^{11}$;
  b) halogen;
  c) $CF_3$;
  d) $SR^{11}$;
  e) $COOR^{12}$;
  f)

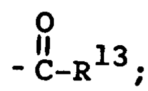

  g) tetrazole;
  h) $-SOR^{11}$;
  i) $-CONR^{12}R^{12}$;
  j) $-SO_2NR^{12}R^{12}$;
  k) $-SO_2R^{11}$;
  l)

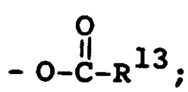

  m) CN;
  n) $N_3$;

$R^6$ is located at position 3' or 4' and is selected from:

(1) alkyl having 1 to 6 carbons;
(2) M wherein M is
  a) $OR^{11}$;
  b) halogen;
  c) $CF_3$;
  d) $SR^{11}$;
  e) $COOR^{12}$;
  f)

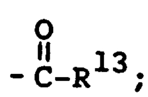

  g) tetrazole;
  h) $-SOR^{11}$;
  i) $-CONR^{12}R^{12}$;
  j) $-SO_2NR^{12}R^{12}$;
  k) $-SO_2R^{11}$;

l)

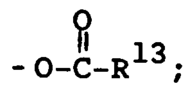

m) CN;
n) $N_3$;
provided that when $R^1$, $R^2$, $R^3$ or $R^4$ is alkyl having 1 to 6 carbons, it is not located at position 6;
each $R^9$ is independently H, or alkyl of 1 to 4 carbons;
$R^{10}$ is lower alkyl,

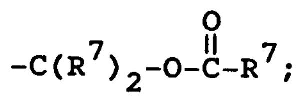

or

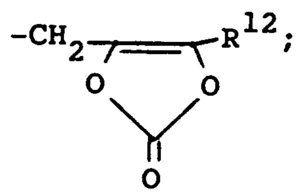

r is 1 and the remaining substituents are as defined initially for the compounds of Formula I.

**Table 1**
**Novel Tetrahydrocarbazole Alkanoic Esters**

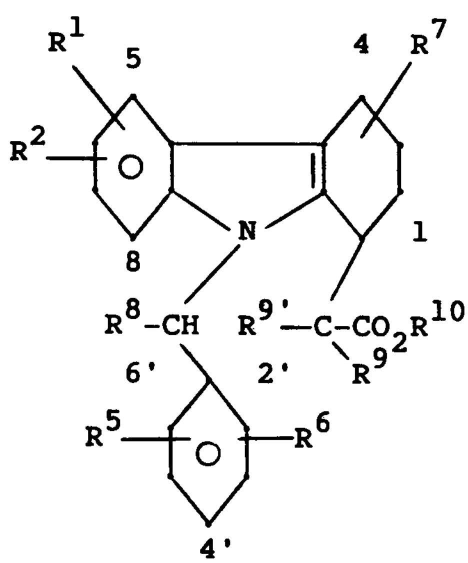

| Compound | $R^1$ | $R^2$ | $R^5$ | $R^6$ | $R^9$, $R^{9'}$ | $R^7$ | $R^8$ | $R^{10}$ |
|---|---|---|---|---|---|---|---|---|
| 1 (Ex.1) | 6-F | H | 4'-Cl | H | H, H | H | H | Et |
| 2 (Ex. 4) | 6-OMe | H | 4'-Cl | H | H, H | H | H | Et |
| 3 (Ex. 18) | 6-F (-) isomer | H | 4'-Cl | H | H, H | H | H | Me |
| 4 (Ex. 19) | 6-F (+) isomer | H | 4'-Cl | H | H, H | H | H | Me |
| 5 (Ex. 5) | 6-F | H | H | H | H, H | H | H | Et |
| 6 (Ex. 6) | 6-F | H | 4'-OMe | H | H, H | H | H | Et |
| 7 (Ex. 7) | 6-F | H | 3'-Cl | 4'-Cl | H, H | H | H | Et |
| 8 (Ex. 8) | 6-F | H | H | H | H, H | H | Me | Et |
| 9 (Ex 9) | H | H | 4'-Cl | H | H, H | H | H | Et |
| 10 (Ex. 10) | 6-Cl | H | 4'-Cl | H | H, H | H | H | Et |
| 11 (Ex. 11) | 8-Me | H | 4'-Cl | H | H, H | H | H | Et |
| 12 (Ex. 12) | 6-Br | H | 4'-Cl | H | H, H | H | H | Et |

| Compound | $R^1$ | $R^2$ | $R^5$ | $R^6$ | $R^9$, $R^{9'}$ | $R^7$ | $R^8$ | $R^{10}$ |
|---|---|---|---|---|---|---|---|---|
| 13 (Ex. 14) | 8-F | H | 4'-Cl | H | H, H | H | H | Et |
| 14 | 6-F | H | 4'-Cl | H | H, H | 3-t-Bu | H | $CH_2C_6H_5$ |
| 15 | 5-F | H | 4'-Cl | H | H, H | H | H | $CH_2OAc$ |
| 16 | 7-F | H | 4'-Cl | H | H, H | H | H | 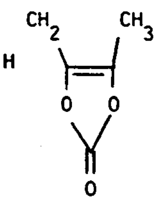 |
| 17 (Ex. 15) | 5-Cl | 7-Cl | 4'-Cl | H | H, H | H | H | Et |
| 18 (Ex. 16) | 6-Cl | 8-Cl | 4'-Cl | H | H, H | H | H | Et |
| 19 | 6-F | H | 4'-Cl | H | Me, H | H | H | 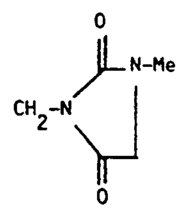 |
| 20 | 6-F | H | 4'-Cl | H | Me, Me | H | H | 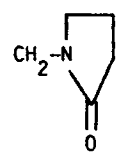 |
| 21 | 6-F | H | 4'-Cl | H | H, H | 1-Me | H | 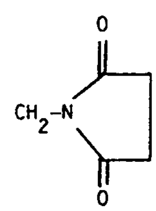 |

| | | | | | | | | | |
|---|---|---|---|---|---|---|---|---|---|
| 22 | (ex 19) | 6-$CF_3$ | H | 4'-Cl | H | H,H | H | H | Et |
| 23 | (ex 20) | 6-SMe | H | 4'-Cl | H | H,H | H | H | Et |
| 24 | (ex 21) | 6-SOMe | H | 4'-Cl | H | H,H | H | H | Et |
| 25 | (ex 22) | 6-$SO_2$Me | H | 4'-Cl | H | H,H | H | H | Et |
| 26 | (ex 23) | 8-CH$(Me)_2$ | H | 4'-Cl | H | H,H | H | H | Et |
| 27 | (ex 24) | 8-SMe | H | 4'-Cl | H | H,H | H | H | Me |
| 28 | (ex 25) | 8-SOMe | H | 4'-Cl | H | H,H | H | H | Me |
| 29 | (ex 26) | 6-F | H | 4'-Cl | H | H,H | 3-Me | H | Et |
| 30 | (ex 27) | 6-F | 8-F | 4'-Cl | H | H,H | H | H | Me |
| 31 | (ex 28) | 6-OMe | 8-Me | 4'-Cl | H | H,H | H | H | Me |
| 32 | (ex 29) | 6-F(-)Isomer | 8-F | 4'-Cl | H | H,H | H | H | Me |
| 33 | (ex 30) | 6-F(+)Isomer | 8-F | 4'-Cl | H | H,H | H | H | Me |
| 34 | (ex 31) | 8-Me(-)Isomer | H | 4'-Cl | H | H,H | H | H | Me |
| 35 | (ex 32) | 8-Me(+)Isomer | H | 4'-Cl | H | H,H | H | H | Me |
| 36 | (ex 33) | 8-F(-)Isomer | H | 4'-Cl | H | H,H | H | H | Me |
| 37 | (ex 34) | 8-F(+)Isomer | H | 4'-Cl | H | H,H | H | H | Me |
| 38 | | 6-F | 8-F | 3'-Cl | 4'-Cl | H,H | H | H | Me |
| 39 | | 6-F | 8-F | 2'-Cl | 4'-Cl | H,H | H | H | Me |
| 40 | | 6-F | 8-F | 4'-OMe | H | H,H | H | H | Me |
| 41 | | 6-F | 8-F | 4'-OH | H | H,H | H | H | Me |
| 42 | | 6-F | 8-F | 4'-SMe | H | H,H | H | H | Me |
| 43 | | 6-F | H | 4'-S(O)Me | H | H,H | H | H | Me |
| 44 | | 6-F | 8-F | 4'-NHCOMe | H | H,H | H | H | Me |
| 45 | | 6-F | H | 4'-$S(O)_2$Me | H | H,H | H | H | Me |
| 46 | | 6-F | H | 4'-F | H | H,H | H | H | Me |
| 47 | | 6-F | H | 4'-Br | H | H,H | H | H | Me |
| 48 | | 6-F | 8-Me | 4'-Cl | H | H,H | H | H | Me |
| 49 | | 6-F | H | 4'-$CO_2$H | H | H,H | H | H | Me |
| 50 | | 6-F | H | 4'-$CO_2$Me | H | H,H | H | H | Me |
| 51 | | 6-F | 8-F | 4'-n-$C_3H_7$ | H | H,H | H | H | Me |
| 52 | | 6-F | 8-F | 3'-I | 4'-OH | H,H | H | —H— | Me |
| 53 | | 6-F | 8-F | 4'-I | H | H,H | H | H | Me |
| 54 | | 6-$N_3$ | H | 4'-Cl | H | H,H | H | H | Me |
| 55 | | 6-F | H | 4'-$N_3$ | H | H,H | H | H | Me |

The following reaction schemes illustrate the preparation of the compounds of the present invention:

Scheme I Preparation of Formula I Compounds

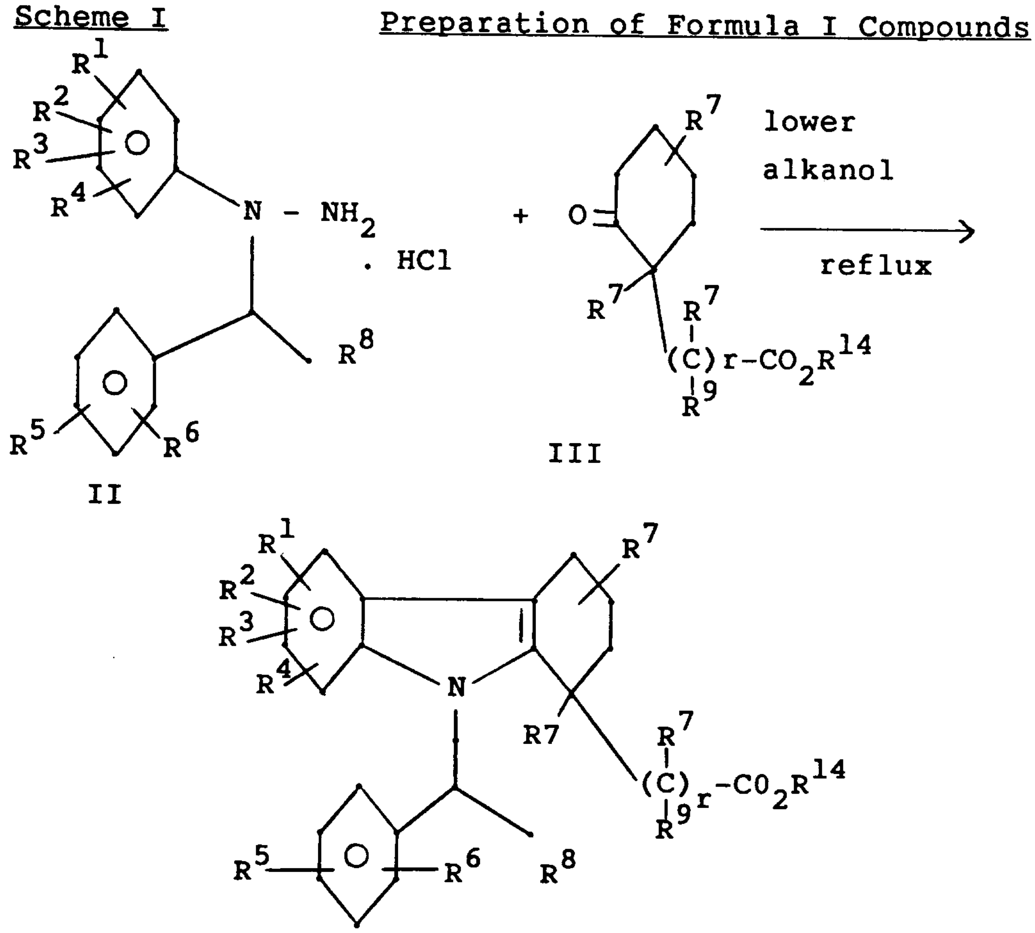


II + III

Ia

The reaction can be conveniently carried out in an alcohol solvent such as t-butanol, i-butanol, i-propanol and the like.

The following ketones (1,2,4) of structure III are known in the art, and ketone 3 is readily prepared by procedures analogous to those for the known ketones.

TABLE 2

Ketones of Formula III

| No. | Structure | Reference |
| --- | --- | --- |
| 1. | 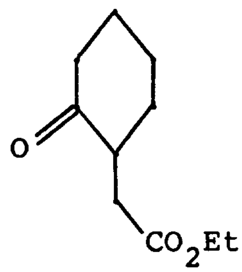 | Ethyl 2-cyclohexanone acetate; commercially available (Aldrich) |
| 2. | 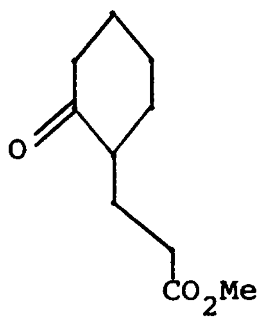 | Methyl 2-cyclohexanone propionate; J.A.C.S. 85 207 (1963) G. Stork, A. Brizzolara, H. Landesman, J. Scmuszkovicz and R. Terrell |
| 3. | 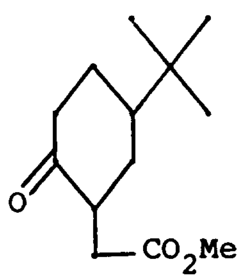 | Methyl 4-t-butyl-2-cyclohexanone acetate |
| 4. | 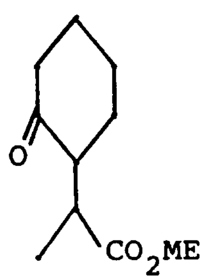 | Methyl 2-(2-cyclohexanone) propionate J.A.C.S. 85 207 (1963) G. Stork, A. Brizzolara, H. Landesman, J. Scmuszkovicz and R. Terrell |

TABLE 2 (continued)

KETONES OF FORMULA III

| No. | Structure | Reference |
|---|---|---|
| 5. | 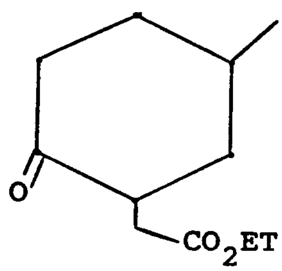 | Ethyl 4-methyl-2-cyclohexanone acetate |

The sequence described above is an application of the Fischer Indole Synthesis. Numerous indole syntheses are described in reviews, such as, for example "Heterocyclic Compounds" Volume 25, Parts I, II, III, W. J. Houlihan (Ed.), Interscience, J. Wiley & Sons, N. Y., 1979. Appropriate manipulations of functional groups using sequences described in such reviews will lead to the compounds of the present invention.

Scheme II Preparation of Hydrazine Derivatives (II)

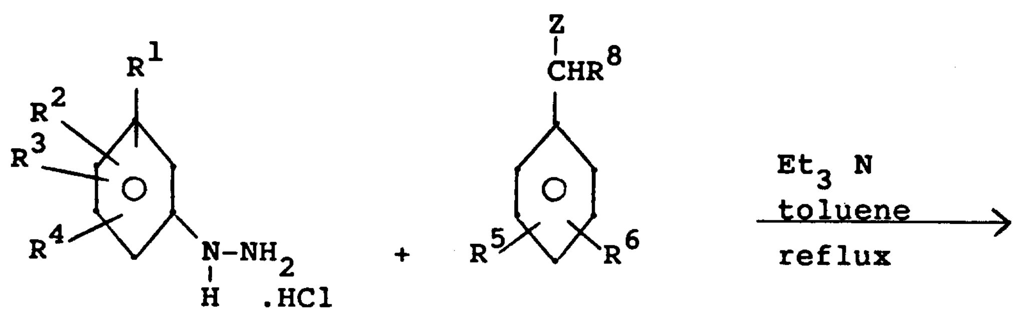


(IV) (V)

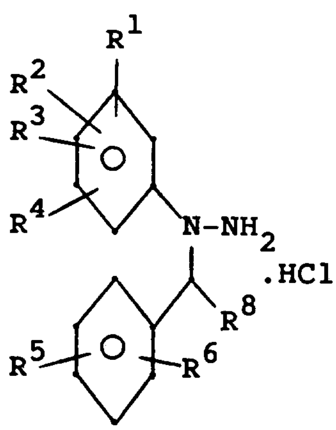

II

Z is a leaving group such as Cl, Br, I, mesylate or tosylate.

With regard to Scheme II, the preparation of hydrazine starting materials is illustrated by the preparation of 1-(4-chlorobenzyl)-1-(4-methoxyphenyl)-hydrazine. A mixture of 10 g of p-methoxyphenylhydrazine hydrochloride, 75 ml of toluene and 11.5 ml of triethylamine was heated at reflux for 60 minutes. Then, 7.1 g of p-chlorobenzyl chloride was added. After stirring 16 hours at reflux, triethylamine hydrochloride was filtered off and washed with ethyl ether. The filtrate and washing were concentrated in vacuo and chromatographed on a silica gel column (hexane-ethyl acetate, 9:1) to give 6.64 g of 1-(4-chlorobenzyl)-1-(4-methoxyphenyl)hydrazine. Other hydrazines, similarly prepared, are also shown in Table 3, below.

TABLE 3

Hydrazines

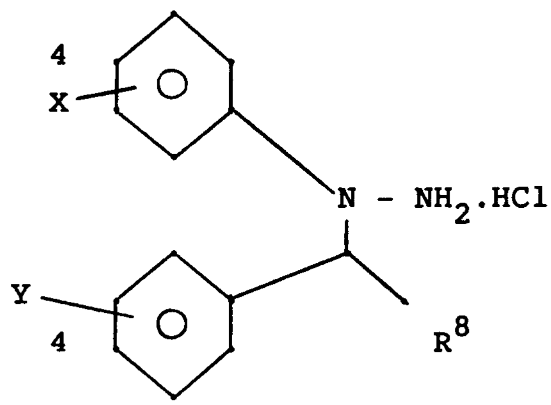

| Compound No. | X | Y | $R^8$ | Compound Name |
|---|---|---|---|---|
| 1. | 4-F | 4-Cl | H | 1-(4-chlorobenzyl)-1-(4-fluorophenyl) hydrazine hydrochloride |
| 2. | 3,5-$Cl_2$ | 4-Cl | H | 1-(4-chlorobenzyl)-1-(3,5-dichlorophenyl)hydrazine hydrochloride |
| 3. | 4-OMe | 4-Cl | H | 1-(4-chlorobenzyl)-1-(4-methoxyphenyl) hydrazine hydrochloride |
| 4. | 2-Me | 4-Cl | H | 1-(4-chlorobenzyl)-1-(2-methylphenyl) hydrazine hydrochloride |

TABLE 3 (Cont'd)

| | | | | |
|---|---|---|---|---|
| 5. | 4-Me | 4-Cl | H | 1-(4-chlorobenzyl)-1-(4-methylphenyl) hydrazine hydrochloride |
| 6. | 4-Cl | 4-Cl | H | 1-(4-chlorobenzyl)-1-(4-chlorophenyl) hydrazine hydrochloride |
| 7. | H | 4-Cl | H | 1-(4-chlorobenzyl)-1-(phenyl) hydrazine hydrochloride |
| 8. | 4-Br | 4-Cl | H | 1-(4-chlorobenzyl)-1-(4-bromophenyl) hydrazine hydrochloride |
| 9. | 3-F | 4-Cl | H | 1-(4-chlorobenzyl)-1-(3-fluorophenyl) hydrazine hydrochloride |
| 10. | 2,4-$Cl_2$ | 4-Cl | H | 1-(4-chlorobenzyl)-1-(2,4-dichlorophenyl)hydrazine hydrochloride |
| 11. | 4-F | H | H | 1-(benzyl)-1-(4-fluorophenyl) hydrazine hydrochloride |
| 12. | 4-F | 4-OMe | H | 1-(4-methoxybenzyl)-1-(4-fluorophenyl) hydrazine hydrochloride |

TABLE 3 (Cont'd)

| | | | | |
|---|---|---|---|---|
| 13. | 4-F | 3,4-$Cl_2$ | H | 1-(3,4-dichlorobenzyl)-1-(4-fluoro-phenyl) hydrazine hydrochloride. |
| 14. | 4-F | H | $CH_3$ | 1-[1-(phenyl)ethyl]-1-(4-fluorophenyl) hydrazine hydrochloride |
| 15. | 2-F | 4-Cl | H | 1-(4-chlorobenzyl)-1-(2-fluorophenyl) hydrazine hydrochloride. |
| 16. | 4-$CH(Me)_2$ | 4-Cl | H | 1-(4-chlorobenzyl)-1-(4-isopropylphenyl)hydrazine hydrochloride |
| 17. | 4-$C(Me)_3$ | 4-Cl | H | 1-(4-chlorobenzyl)-1-(4-tert-butylphenyl)hydrazine)hydrochloride |
| 18. | 4-$CF_3$ | 4-Cl | H | 1-(4-chlorobenzyl)-1-(4-trifluoromethylphenyl)hydrazine hydrochloride |
| 19. | 4-SMe | 4-Cl | H | 1-(4-chlorobenzyl)-1-(4-methylthiophenyl)hydrazine hydrochloride |
| 20. | 2-$CH(Me)_2$ | 4-Cl | H | 1-(4-chlorobenzyl)-1-(2-isopropylphenyl)hydrazine hydrochloride |

To prepare certain esters representative of the Formula I compounds, it may be advantageous to first prepare the lower alkyl esters as illustrated in Scheme I. Hydrolysis of these lower alkyl esters by conventional means, such as by using NaOH or KOH in aqueous ethanol, followed by acidification, then produces the corresponding carboxylic acids VI, as illustrated in Scheme III. The carboxylic acid is then reacted with an alkylating agent, $R^{10}$-Z, in the presence of a suitable base and solvent combination, such as for example, $Na_2CO_3$ in acetone or triethylamine in dimethylformamide, to produce the esters Ib. Compounds 15, 16, 17, 20, 21 and 22 of Table I are conveniently prepared by this method.

Another method of preparing the esters of Formula I from the acid, VI consists of treating the latter with a diazoalkane (such as diazomethane) in a suitable non-reactive solvent such as ether or methanol to obtain an ester Ic. Other methods are shown in Ogliaruso and Wolfe in Patai, The Chemistry of Acid Derivatives, Supplement B, Wiley, New York, 1979, pp. 411-436) which is hereby incorporated by reference.

**Scheme III Preparation of Formula I Compounds**

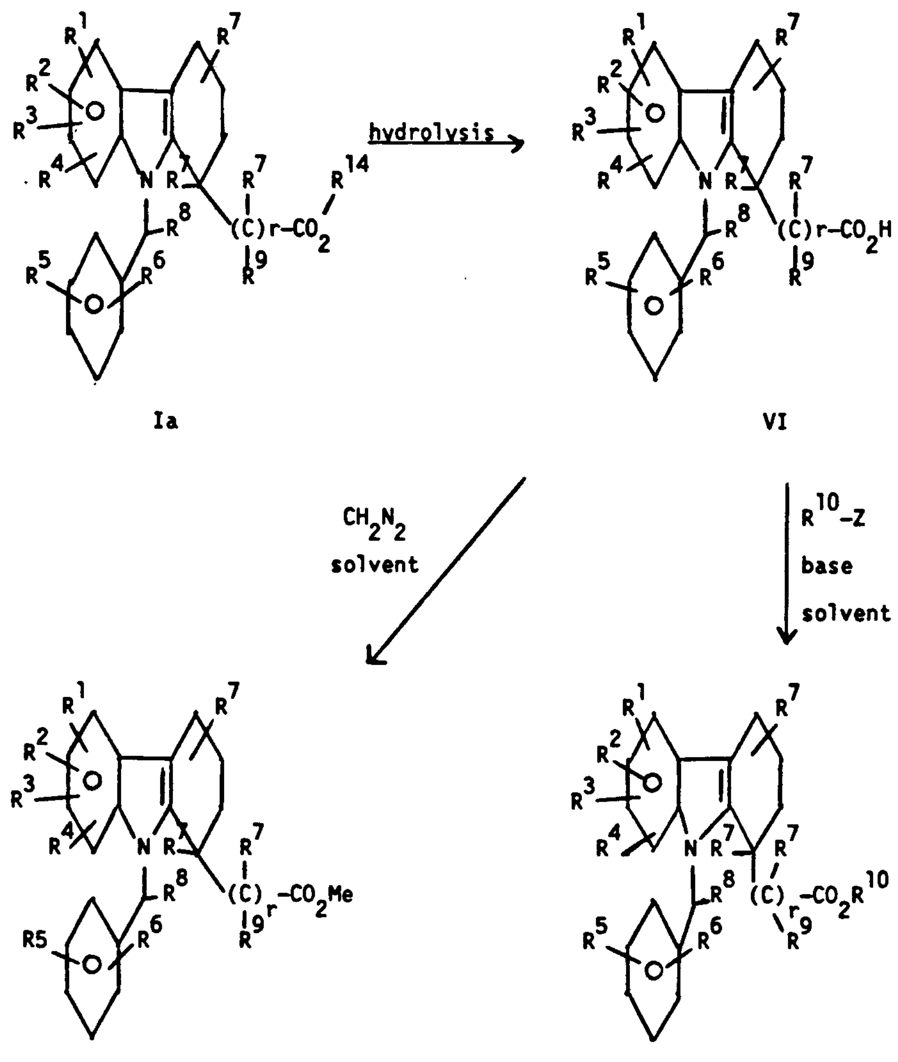


Ia, VI, Ic, Ib

In those instances when asymmetric centers are present, more than one stereoisomer is possible, and all possible isomeric forms are deemed to be included within the planar structural representations shown. Optically active (R) and (S) isomers may be resolved using conventional techniques known to the skilled artisan.

Among the resolved isomers in Table 1, the esters of the minus (-) acids, compounds 3,36,38 and 40 are preferred.

Scheme IV Alternative Preparation of Formula I Compounds

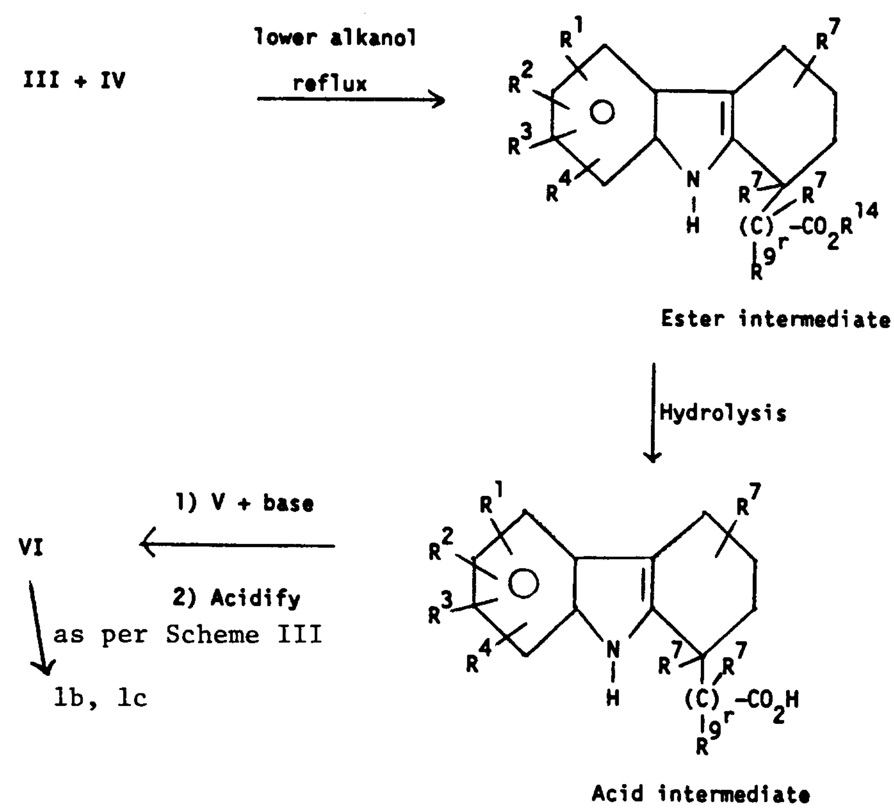


Scheme IV illustrates an alternative synthesis of the compounds of Formula I. In this Scheme a Fischer indole synthesis is carried out using a phenylhydrazine IV and the ketone III, followed by hydrolysis. The acid intermediate is then N-benzylated with the reagent V, preferably using a strong base such as potassium t-butoxide to effect the reaction. Acidification of the reaction mixture then yields the acid VI which can be converted to compounds of Formula I as indicated in Scheme III.

**Scheme V** **Preparation of Sulfoxides and Sulfones of Formula I compounds**

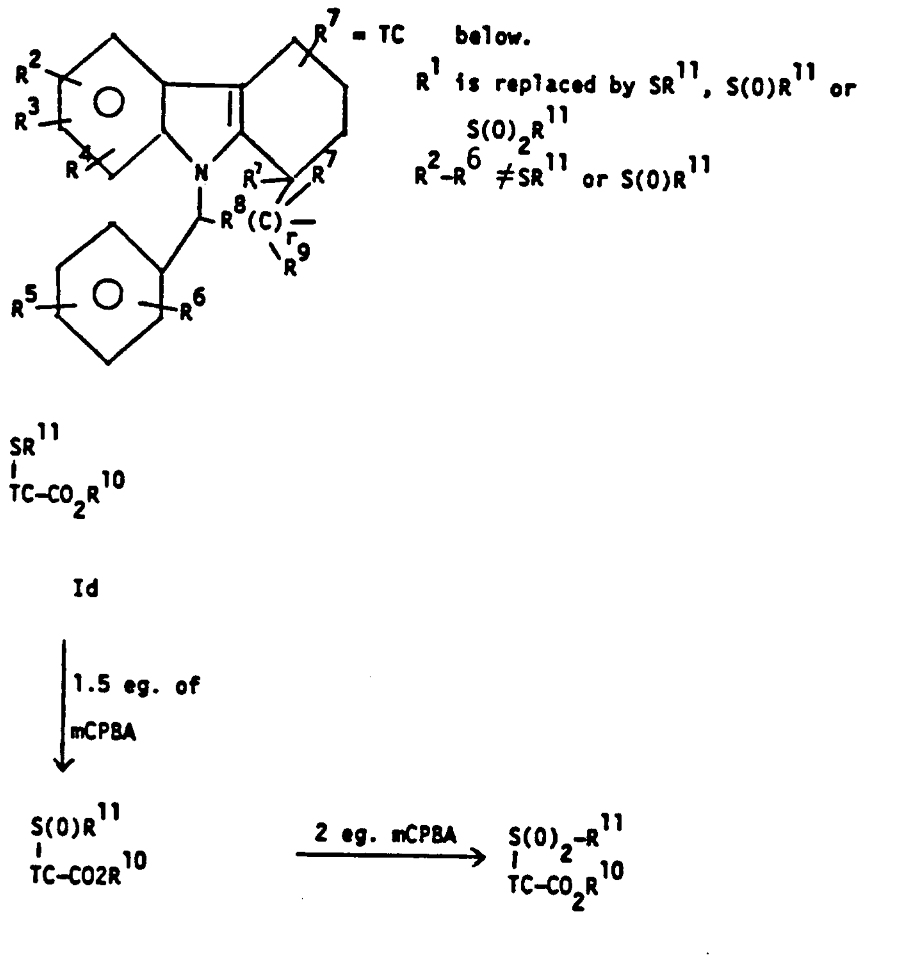


Id

Ie

If

In Scheme V is illustrated a method of preparing derivatives of Formula I in which one of the substituents among $R^1$-$R^4$ is a sulfoxide or a sulfone. It will be obvious to one skilled in the art that a sulfoxide or sulfone derivative of $R^5$ or $R^6$ could be prepared in the same way.

Ester Id (a representative of I) is prepared according to Scheme I or Scheme III. Treatment of Id with a limited amount of an oxidizing agent such as m-chloro-perbenzoic acid yields the sulfoxide ester Ie. Further treatment of Ie with the oxidizing agent, or treatment of Id with an excess (>2 eq.) of the oxidizing agent yields the sulfone ester If.

**Scheme VI** **Preparation of Hydrazine Deniatives IV**

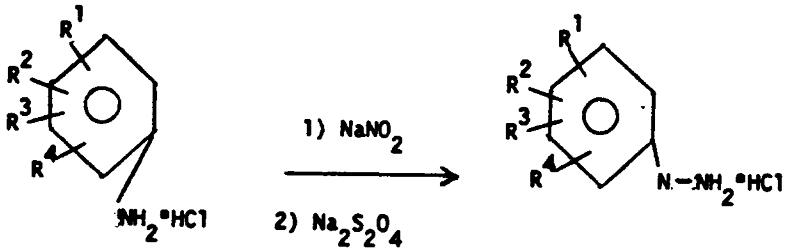


IV

With regard to Scheme VI, the preparation of hydrazine starting materials is illustrated by preparation of 4-methylthiophenyl hydrazine hydrochloride. 4-Methylthioaniline (13.9 g) was added dropwise to cold HCl (6N) (50 mL) and stirred for 5 min in an ice bath. A solution of $NaNO_2$ in water (7.25 g, 15 mL) was then added dropwise and stirred for 15 min. The cold diazonium salt was then cannulated into a stirred cold solution of $Na_2S_2O_4$ in water (50 g, 250 mL). After 20 min, ether (200 mL) was added and the reaction mixture basified with NaOH(10N). The ether layer was decanted, washed with brine, dried over $Na_2SO_4$ and HCl gas was passed through the ether solution to form the hydrochloride salt which precipitated out. After filtration, there was obtained 7.0 g of pure final product. Other hydrazines, similarly prepared, are also shown in Table 4, below.

**TABLE 4**

**HYDRAZINES**

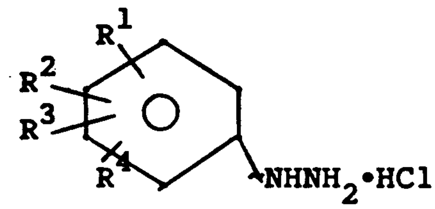

**IV**

| No. | $R^1$ | $R^2$ | Compound Name |
|---|---|---|---|
| 1 | 4-SMe | H | 4-methylthiophenyl hydrazine hydrochloride |
| 2 | 2-CH(Me)$_2$ | H | 2-isopropylphenyl hydrazine hydrochloride |
| 3 | 2-SMe | H | 2-methylthiophenyl hydrazine hydrochloride |
| 4 | 2-Me | 4-Me | 2,4-dimethylphenyl hydrazine hydrochloride |
| 5 | 2-Me | 4-OMe | 4-methoxy-2-methyl-phenyl hydrazine hydrochloride |

$R^3=R^4=H$

The prostaglandin antagonist properties of the compositions and compounds of the present invention can be demonstrated by the biological assay described below.

Agonist-induced Bronchonconstriction in Anesthetized Guinea Pigs (Konzett-Rossler)

Male Hartley strain guinea-pigs (350-500 g) are anesthetized wtih urethane (1.5 g/kg i.p.), given succinylcholine chloride (5 mg/kg s.c.) to suppress spontaneous respiration and artificially ventilated at 60 breaths/minute. Changes in insufflation pressure (resistance to inflation) are measured using a Statham PM-5E differential pressure transducer and recorded on a Beckman Type R-Dynograph. Increases in insufflation pressure are induced at 20 minute intervals with bolus intravenous injections of arachidonic acid (0.5 mg/kg), or U-44069 (15(S)-hydroxy-9α, 11α-(epoxymethano)-prosta-5-cis, 13-trans-dienoic acid) (a stable prostaglandin endoperoxide analogue) (2 μg/kg). After obtaining at least two reproducible control responses to the agonist under study, the antagonist activity of the test compound is determined. In order to assess antagonist activity, the test compound (in solution or suspension) or drug vehicle (1 ml/kg) is administered intravenously in cumulative doses 5 minutes prior to each subsequent agonist challenge. Reductions of the agonist response are calculated as a percent of the control response which immediately preceded the initial antagonist or vehicle dose. $ED_{50}$ values (dose required to inhibit the insufflation pressure increase by 50 percent) are calculated by regression analysis.

In a variation of the assay, the test compound is administered as a single dose intraduodenally (injected into the duodenum which has been previously exposed through a ventral mid-line incision) 10 minutes before challenge with arachidonic acid (0.5 mg/kg) or U-44069 (2 μg/kg). The agonist challenge is continued 10 minutes after administration of the antagonist and every 20 minutes thereafter for a period of up to 2 hours. Reduction of the post drug response is calculated as a percent of the pre-drug control response. This variation of the assay also permits the evaluation of antagonists with a delayed onset of action.

Compounds of Formula I can be tested using the following assay to determine their mammalian leukotriene biosynthesis inhibiting activity.

Rat Peritoneal Polymorphonuclear (PMN) Leukocyte Assay

Rats under ether anesthesia are injected (i.p.) with 8 ml of a suspension of sodium caseinate (6 grams in ca. 50 ml water). After 15-24 hr. the rats are sacrificed ($CO_2$) and the cells from the peritoneal cavity are recovered by lavage with 20 ml of buffer (Eagles MEM containing 30 mM HEPES adjusted to pH 7.4 with NaOH). The cells are pelleted (350 x g, 5 min.), resuspended in buffer with vigorous shaking, filtered, through lens paper, recentrifuged and finally suspended in buffer at a concentration of 10 cells/ml. A 500 μl aliquot of PWN suspension and test compound are preincubated for 2 minutes at 37°C, followed by the addition of 10 μM A-23187. The suspension is stirred for an additional 4 minutes then bioassayed for $LTB_4$ content by adding an aliquot to a second 500 μl portion of the PMN at 37°C. The $LTB_4$ produced in the first incubation causes aggregation of the second PMN, which is measured as a change in light transmission. The size of the assay aliquot is chosen to give a submaximal transmission change (usually -70%) for the untreated control. The percentage inhibition of $LTB_4$ formation is calculated from the ratio of transmission change in the sample to the transmission change in the compound-free control.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastro-intestinal tract, animal studies show that cyto-protective compounds will prevent gastric lesions induced by oral administration of strong acids, strong bases, ethanol, hypertonic saline solutions and the like.

Two assays can be used to measure cyto-protective ability. These assays are; (A) an ethanol-induced lesion assay and (B) an indomethacin-induced ulcer assay and are described in EP 140,684.

The magnitude of a prophylactic or therapeutic dose of a compound of Formula I will, of course, vary with the nature or the severity of the condition to be treated and with the particular compound of Formula I and its route of administration. In general, the daily dose range for anti-asthmatic, anti-allergic, or anti-thrombotic use lies within the range of from about 0.01 mg to about 100 mg per kg body weight of a mammal.

The exact amount of a compound of Formula I to be used as a cytoprotective agent will depend on, inter alia, whether it is being administered to heal damaged cells or to avoid future damage, on the nature of the damaged cells (e.g., gastro-intestinal ulcerations vs. nephrotic necrosis), and on the nature of the causative agent. An example of use of a compound of Formula I to avoid future damage is co-administration with a non-steroidal anti-inflammatory drug (for example, indomethacin).

The effective daily dosage level for compounds of Formula I inducing cytoprotection in mammals, especially humans, will generally range from about 0.002 mg/kg to about 100 mg/kg, preferably from about

0.02 mg/kg to about 30 mg/kg. The dosage may be administered in single or divided individual doses.

Any suitable route of administration may be employed for providing a mammal, especially a human with an effective dosage of a compound of Formula I. For example, oral, rectal, topical, parenteral, ocular, nasal, buccal, intravenous and the like may be employed. Dosage forms include tablets, troches, dispersions, suspensions, solutions, capsules, creams, ointments, aerosols and the like.

The pharmaceutical compositions of the present invention comprise a compound of Formula I as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. The compositions include compositions suitable for oral, rectal, ophthalmic, pulmonary, nasal, dermal, topical or parenteral (including subcutaneous, intramuscular and intravenous) administration, although the most suitable route in any given case will depend on the nature and severity of the conditions being treated and on the nature of the active ingredient. They may be conveniently presented in unit dosage form and prepared by any of the methods well-known in the art of pharmacy.

For use where a composition for intravenous administration is employed, a suitable dosage range for anti-asthmatic, or anti-allergic use is from about 0.01 mg to about 20 mg (preferably from about 0.1 mg to about 10 mg) of a compound of Formula I per kg of body weight per day and for cytoprotective use from about 0.002 mg to about 100 mg (preferably from about 0.02 mg to about 30 mg and more preferably from about 0.1 mg to about 10 mg) of a compound of Formula I per kg of body weight per day. In the case where an oral composition is employed, a suitable dosage range for anti-asthmatic, or anti-allergic use is, e.g. from about 1 to about 100 mg of a compound of Formula I per kg of body weight per day, preferably from about 5 mg to about 40 mg per kg and for cyto-protective use from about 0.01 mg to about 100 mg (preferably from about 0.1 mg to about 30mg and were preferably from about 0.1 mg to about 10 mg) of a compound of Formula I per kg of body weight per day.

For administration by inhalation, the compounds of the present invention are conveniently delivered in the form of an aerosol spray presentation from pressurized packs or a nebuliser, or a powder which may be formulated as a cartridge from which the powder composition may be inhaled with the aid of a suitable device. The preferred delivery system for inhalation in a metered dose inhalation (MDI) aerosol, which may be formulated as a suspension or solution in fluorocarbon propellants.

Suitable topical formulations of compound I include transdermal devices, aerosols, creams, ointments, lotions, dusting powder, and the like.

In practical use, a compound of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the compounds of Formula I may also be administered by controlled release means and/or delivery devices such as those described in U.S. Patent Nos. 3,845,770; 3,916,899; 3,536,809; 3,598,123; 3,630,200 and 4,008,719, the disclosure of which is hereby incorporated herein by reference.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert

diluent, surface active or dispersing agent. Molded tablets may be made by molding in a suitable machine, a mixture of the powdered compound moistened with an inert liquid diluent. Desirably, each tablet contains from about 2.5 mg to about 500 mg of the active ingredient and each cachet or capsule contains from about 2.5 to about 500 mg of the active ingredient.

The following are examples of representative pharmaceutical dosage forms for the compounds of Formula I:

| Injectable Suspension (I.M.) | mg/ml |
|---|---|
| Compound of Formula I | 2.0 |
| Methylcellulose | 5.0 |
| Tween 80 | 0.5 |
| Benzyl alcohol | 9.0 |
| Benzalkonium chloride | 1.0 |
| Water for injection to a total volume of 1 ml | |

| Tablet | mg/tablet |
|---|---|
| Compound of Formula I | 25.0 |
| Microcrystalline Cellulose | 415.0 |
| Providone | 14.0 |
| Pregelatinized Starch | 43.5 |
| Magnesium Stearate | 2.5 |
| | 500 |

| Capsule | mg/capsule |
|---|---|
| Compound of Formula I | 25.0 |
| Lactose Powder | 573.5 |
| Magnesium Stearate | 1.5 |
| | 600 |

In addition to the compounds of Formula I, the pharmaceutical compositions of the present invention can also contain other active ingredients, such as non-steroidal anti-inflammatory drugs (NSAIDs), peripheral analgesic agents such as zomepirac, diflunisal and the like, cyclooxygenase inhibitors, leukotriene antagonists, leukotriene biosynthesis inhibitors, $H_2$-receptor antagonists, antihistaminic agents, prostaglandin antagonists, ACE inhibitors, and thromboxane synthetase inhibitors. The weight ratio of the compound of the Formula I to the second active ingredient may be varied and will depend upon the effective dose of each ingredient. Generally, an effective dose of each will be used. Thus, for example, when a compound of the Formula I is combined with a second active ingredient the weight ratio of the compound of the Formula I to the second ingredient will generally range from about 1000:1 to about 1:1000, preferably from 200:1 to 1:200. Combinations of a compound of the Formula I and other active ingredients will generally be within the aforementioned range, but in each case, an effective dose of each active ingredient should be used.

NSAIDs can be characterized into five groups:

(1) the propionic acid derivatives;

(2) the acetic acid derivatives;

(3) the fenamic acid derivatives;

(4) the biphenylcarboxylic acid derivatives; and

(5) the oxicams

or a pharmaceutically acceptable salt thereof. NSAIDS which are within the scope of this invention are those disclosed in EP 140,684.

Pharmaceutical compositions comprising the Formula I compounds may also contain other inhibitors of the biosynthesis of the leukotrienes such as are disclosed in EP 138,481 (April 24, 1985), EP 115,394 (August 8, 1984), EP 136,893 (April 10, 1985), and EP 140,709 (May 8, 1985), which are hereby

incorporated herein by reference.

The compounds of the Formula I may also be used in combination with leukotriene antagonists such as those disclosed in EP 106,565 (April 25, 1984) and EP 104,885 (April 4, 1984), which are hereby incorporated herein by reference and others known in the art such as those disclosed in European Patent Application Nos. 56,172 and 61,800; and in U.K. Patent Specification No. 2,058,785, which are hereby incorporated herein by reference.

Pharmaceutical compositions comprising the Formula I compounds may also contain as the second active ingredient, other prostaglandin antagonists such as those disclosed in European Patent Application 11,067 (May 28, 1980) or other thromboxane antagonists such as those disclosed in U.S. 4,237,160. They may also contain histidine decarboxyase inhibitors such as $\alpha$-fluoromethyl-histidine, described in U.S. 4,325,961. The compounds of the Formula I may also be advantageously combined with an $H_1$ or $H_2$-receptor antagonist, such as for instance benadryl, dramamine, histadyl, phenergan, terfenadine, acetamazole, cimetidine, ranitidine, famotidine, aminothiadiazoles disclosed in EP 40,696 (December 2, 1981) and like compounds, such as those disclosed in U.S. Patent Nos. 4,283,408; 4,362,736; 4,394,508; and a pending application, U.S. S.N. 301,616, filed September 14, 1981. The pharmaceutical compositions may also contain a $K^+/H^+$ ATPase inhibitor such as omeprazole, disclosed in U.S. Pat. 4,255,431, and the like. Compounds of I may also be usefully combined with most cell stabilizing agents, such as 1,3-bis(2-carboxychromon-5-yloxy)-2-hydroxypropane and related compounds described in British Patent Specifications 1,144,905 and 1,144,906. Another useful pharmaceutical composition comprises the Formula I compounds in combination with serotonin antagonists such as methysergide, the serotonin antagonists described in Nature, Vol. 316, pages 126-131, 1985, and the like. Each of the references referred to in this paragraph is hereby incorporated herein by reference.

When the second active ingredient in compositions of this invention is a thromboxane synthetase inhibitor, such inhibitor can be as described in UK 2,038,821 (e.g., UK 37248 and dazoxiben hydrochloride), U.S.P. 4,217,357 (e.g., UK 34787), U.S.P. 4,444,775 (e.g., CGS 13080), U.S.P. 4,226,878 (e.g., ONO 046), U.S.P. 4,495,357 (e.g., U63557A) U.S.P. 4,273,782 (e.g., UK-38485), or EP 98,690 (e.g., CV-4151).

An embodiment of the invention is a cardiovascular composition useful for treating arterial thrombosis which comprises an antithrombotic compound of the Formula I.

A further embodiment of the invention is a cardiovascular composition useful for treating arterial thrombosis which comprises: (1) the antithrombotic Formula I compound defined above; and, (ii) an angiotensin converting enzyme (ACE) inhibitor compound which is a member of the group: carboxyalkyl dipeptide derivatives; captopril [1-(3-mercapto-2-methyl-1-oxopropyl)-L-proline]; 2-[N-(S)-1-ethoxy-carbonyl-3-phenylpropyl)-S-alanyl]-cis,endo-2-azabicyclo[3,3,0]octane-3(S)-carboxylic acid; N-((S)-1-ethoxycarbonyl-3-phenylpropyl)-L-alanyl-N-(2-indanyl)-glycine; 1-(N-[(S)-1-ethoxy-carbonyl-3-phenylpropyl]-L-alanyl)-cis,syn-octahydro-(H-indole-2-S)-carboxylic acid; 2-(N-[(S)-1-ethoxy-carbonyl-3-phenylpropyl]-L-alanyl)-1,2,3,4-tetrahydro-iso-isoquinoline-3(S)-carboxylic acid; and, 1-carboxy-methyl-3(S)-(1(S)-ethoxycarbonyl-3-phenylpropylamino)-2,3,4,5-tetrahydro1H[1]-benzazepine-2-one.

In particular the class of ACE inhibitors which have been found to have a potentiating effect when used in combination with the Formula I compounds are those disclosed in U.S. Patent 4.374,829, which also discloses methods for their preparation and which patent is incorporated herein by reference. Of the carboxyalkyl dipeptides disclosed in U.S. Patent 4,374,829, those of particular interest in this invention are N-[1(S)-ethoxycarbonyl-3-phenylpropyl]-L-alanyl-L-proline, also known and referred to herein as enalapril; N-[1(S)-carboxy-3-phenylpropyl]-L-alanyl-L-proline, also know and referred to herein as enalapril diacid; and, N$\alpha$-[1(S)-carboxy-3-phenylpropyl]-L-lysyl-L-proline, also known and referred to herein as lisinapril.

The combination composition of the invention can contain varying amounts of (i) the Formula I antithrombotic compound and (ii) ACE inhibitor antihypertensive compounds. The weight ratio of (i):(ii) can range from about 25 to 1; preferably from about 10 to 1. In addition to the active ingredients of (i) alone or of (i) and (ii) in combination, the compositions of the invention can also contain other conventional pharmaceutically acceptable compounding ingredients, as necessary or desired. Such ingredients are generally referred to as carriers or diluents. Conventional procedures for preparing such compositions in appropriate dosage forms can be utilized. Whatever the dosage form, it will contain a pharmaceutically effective amount of the present composition.

The combination compositions can be administered orally or other than orally; e.g., parenterally, by insufflation, topically, rectally, etc.; using appropriate dosage forms; e.g., tablets, capsules, suspensions, solutions, and the like, for oral administration; suspension emulsions, and the like, for parenteral administration; solutions for intravenous administration; and ointments, transdermal patches, and the like, for topical administration. These compositions are formulated similarly to the compositions discussed above.

Treatment dosage for human beings for cardiovascular use can be varied as necessary. Generally, daily dosages of the composition of the invention can range from about 6000 to about 10 mg; preferably, from about 3000 to about 20 mg.

The amount of active ingredient that may be combined with the carrier materials to produce a single dosage form for cardiovascular use will vary depending upon the host treated and the particular mode of administration. For example, a formulation intended for oral administration may contain from 5 mg to 5 gm of active agents compounded with an appropriate and convenient amount of carrier material which may vary from about 5 to about 95 percent of the total composition. Dosage unit forms will generally contain between from about 20 mg to about 500 mg of active ingredients.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The composition of this invention inhibits platelet accumulation at the damaged endothelial surface via the Formula I compound. This inhibitory effect is potentiated by the presence of the antihypertensive compound.

Thus, the compositions of the invention are useful in treating thrombosis and are also of value in the management of acute and chronic congestive heart failure, and limitation of myocardial infarct damage.

In vivo testing of the composition of this invention in test animals (rabbits) can be used to demonstrate that this composition is pharmaceutically effective in decreasing platelet-related arterial thrombic formation.

To demonstrate the potentiation of the antihypertensive compound on the anti-thrombotic Formula I compound comprising the combination composition of the invention, the effect of these compounds on test animals (rabbits) can be determined separately and then in combination. The effect of a different class of antihypertensive agents singly and in combination with the Formula I compound of the invention can also be determined for comparative purposes. The methods employed are described in U.S. Patent 4,558,037 which is hereby incorporated by reference.

The following examples illustrate the preparation of the compounds of the present invention without, however, limiting the same thereto.

All temperature are in degrees Celsius.

## Example 1

### Ethyl 9-p-chlorobenzyl-6-fluoro-1,2,3,4-tetrahydrocarbazol-1-yl-acetate

To 3.50 g of 1-(4-chlorobenzyl)-1-(4-fluorophenyl)hydrazine hydrochloride in 70 cc of iso-propanol was added 2.23 g of ethyl 2-cyclohexanone acetate. The reaction was refluxed under nitrogen for 16 hours. The resulting reaction mixture was then evaporated to dryness and the residue suspended in ether. The solid material was then filtered. The ether filtrate was washed with water, dried and evaporated. The resulting syrup was chromatographed on silica gel to give 2.8 g (42%) of the title compound.

$^1H$ NMR δ : 1.25 (t, 3H, $CO_2CH_2CH_3$); 1.80-2.00 (m, 4H); 2.35-2.85 (m, 4H); 3.38 (m, 1H); 4.10 (q, 2H, $CO_2CH_2CH_3$); 5.28 (2d, 2H, Ar $CH_2$); 6.80-7.30 (m, 7H, Ar).

## Example 2

### Methyl 3-(9-p-chlorobenzyl-6-fluoro-1,2,3,4-tetrahydrocarbazol-1-yl)-propanoate

Following the procedure of Example 1, but using 1-(4-chlorobenzyl)-1-(4-fluorophenyl)hydrazine hydrochloride and methyl 2-cyclohexanone propionate as starting materials, the title compound is prepared.

## Example 3

### Methyl 3-(9-p-chlorobenzyl-6-methoxy-1,2,3,4-tetrahydrocarbazol-1-yl)-propanote

Following the procedure of Example 1, but using 1-(4-chlorobenzyl)-1-(4-methoxyphenyl)hydrazine hydrochloride and methyl 2-cyclohexanone propionate as starting materials, the title compound is prepared.

Example 4

Ethyl 9-p-chlorobenzyl-6-methoxy-1,2,3,4-tetrahydrocarbazol-1-yl-acetate

Following the procedure of Example 1, but using 1-(4-chlorobenzyl)-1-(4-methoxyphenyl)hydrazine hydrochloride and ethyl 2-cyclohexanone acetate as starting materials, the title compound is prepared.

Example 5

Ethyl 9-benzyl-6-fluoro-1,2,3,4-tetrahydrocarbazol-1-yl-acetate

Following the procedure of Example 1, but using 1-(benzyl)-1-(4-fluorophenyl)hydrazine hydrochloride and ethyl 2-cyclohexanone acetate as starting materials, the title compound was prepared.

$^1$HNMR δ : 1.15 (t, 3H, $CO_2CH_2CH_3$);1.80-2.00 (m, 4H); 2.38-2.85 (m, 4H); 3.45 (m, 1H); 4.10 (q, 2H, $CO_2CH_2CH_3$); 5.30 (2d, 2H, Ar $CH_2$); 6.78-7.25 (m, 8H, Ar).

Example 6

Ethyl 9-p-methoxybenzyl-6-fluoro-1,2,3,4-tetrahydrocarbazol-1-yl-acetate

Following the procedure of Example 1, but using 1-(4-methoxybenzyl)-1-(4-fluorophenyl)hydrazine hydrochloride and ethyl 2-cyclohexanone acetate as starting materials, the title compound was prepared.

$^1$H NMR δ : 1.15 (t, 3H, $CO_2$,$CH_2CH_3$);1.80-1.95 (m, 4H); 2.40-2.85 (m, 4H); 3.63 (m, 1H); 3.75 (s, 3H, $OCH_3$); 4.13 (q, 2H, $CO_2CH_2CH_3$); 5.25 (2d, 2H, Ar $CH_2$); 6.75-7.25 (m, 7H, Ar).

Example 7

Ethyl 9-(3,4-dichloro)benzyl-6-fluoro-1,2,3,4-tetrahydrocarbazol-1-yl-acetate

Following the procedure of Example 1, but using 1-(3,4-dichlorobenzyl)-1-(4-fluorophenyl) hydrazine hydrochloride and ethyl 2-cyclohexanone acetate as starting materials, the title compound was prepared.

$^1$H NMR δ : 1.15 (t, 3H, $CO_2CH_2CH_3$);1.80-2.00 (m, 4H); 2.30-2.85 (m, 4H); 3.35 (m, 1H); 4.15 (q, 2H, $CO_2CH_2CH_3$); 5.25 (2d, 2H, Ar $CH_2$); 6.70-7.45 (m, 6H, Ar).

Example 8

Ethyl 9-[1-(1-phenyl)ethyl]-6-fluoro-1,2,3,4-tetrahydrocarbazol-1-yl-acetate

Following the procedure of Example 1, but using 1-[1-(1-phenyl)ethyl]-1-(4-fluorophenyl)-hydrazine hydrochloride and ethyl 2-cyclohexanone acetate as starting materials, the title compound was prepared.

H NMR δ : 1.15 (t, 3H, $CO_2CH_2CH_3$);1.80-2.00 (m, 4H); 2.05 (d, 3H, Ar - CH - Me); 2.50-2.85 (m, 4H); 4.55 (m, 1H); 4.20 (Q, 2H, $CO_2CH_2CH_3$); 5.65 (q, 1H, Ar - CH-$CH_3$); 6.60-7.40 (m, 8H, Ar).

Example 9

Ethyl 9-p-chlorobenzyl-1,2,3,4-tetrahydrocarbazol-1-yl-acetate

Following the procedure of Example 1, but using 1-(4-chlorobenzyl)-1-(phenyl)hydrazine hydrochloride and ethyl 2-cyclohexanone acetate as starting materials, the title compound was prepared.

H NMR δ : 1.25 (t, 3H, $CO_2CH_2CH_3$);1.80-2.00 (m, 4H); 2.38-2.88 (m, 4H); 3,40 (m, 1H); 4.15 (q, 2H, $CO_2CH_2CH_3$); 4.30 (2d, 2H, Ar $CH_2$); 6.85-7.55 (m, 8H, Ar).

Example 10

Ethyl 9-p-chlorobenzyl-6-chloro-1,2,3,4-tetrahydrocarbazol-1-yl-acetate

Following the procedure of Example 1, but using 1-(4-chlorobenzyl)-1-(4-chlorophenyl)hydrazine hydrochloride and ethyl 2-cyclohexanone acetate as stating materials, the title compound was prepared.

H NMR δ : 1.25 (t, 3H, $CO_2CH_2CH_3$);1.80-2.00 (m, 4H); 2.35-2.85 (m, 4H); 3.40 (m, 1H); 4.15 (q, 2H, Ar $CH_2$); 6.82-7.50 (m, 7H, Ar).

Example 11

Ethyl 9-p-chlorobenzyl-8-methyl-1,2,3,4-tetrahydrocarbazol-1-yl-acetate

Following the procedure of Example 1, but using 1-(4-chlorobenzyl)-1-(2-methylphenyl)hydrazine hydrochloride and ethyl 2-cyclohexanone acetate as starting materials, the title compound was prepared.

$^1$H NMR δ : 1.30 (t, 3H, $CO_2CH_2CH_3$); 2.00-2.25 (m, 4H); 2.60-3.15 (m, 4H); 3.58 (m, 1H); 4.40 (q, 2H, $CO_2CH_2CH_3$); 5.78 (2d, 2H, Ar $CH_2$); 6.95-7.65 (m, 7H, Ar).

Example 12

Ethyl 6-bromo-9-p-chlorobenzyl-1,2,3,4-tetrahydrocarbazol-1-yl-acetate

Following the procedure of Example 1, but using 1-(4-chlorobenzyl)-1-(4-bromophenyl)hydrazine hydrochloride and ethyl 2-cyclohexanone acetate as starting materials, the title compound is prepared.

Example 13

Methyl 2-(9-p-chlorobenzyl-6-fluoro-1,2,3,4-tetrahydrocarbazol-1-yl)-propanoate

Following the procedure of Example 1, but using 1-(4-chlorobenzyl)-1-(4-fluorophenyl)hydrazine hydrochloride and methyl 2-(2-cyclohexanone) propionate as starting materials, the title compound was prepared.

$^1$H NMR δ : 0.98 (d, 3H, $CH_3$-CH-$CO_2$ Me); 1.72-2.00 (m, 4H); 2.60-2.85 (m, 3H); 3.35 (m, 1H); 3.64 (s, 3H, $CO_2$Me); 5.27 (2d, 2H, Ar-$CH_2$); 6.75-7.25 (m, 7H, Ar).

Example 14

Ethyl 9-p-chlorobenzyl-8-fluoro-1,2,3,4-tetrahydrocarbazol-1-yl-acetate

Following the procedure of Example 1, but using 1-(4-chlorobenzyl)-1-(2-fluorophenyl)hydrazine hydrochloride and ethyl 2-cyclohexanone acetate as starting materials, the title compound was prepared.

$^1$H NMR δ : 1.25 (t, 3H, $CO_2$ $CH_2$ $CH_3$);1.50-2.00 (m, 4H); 2.38-2.95 (m, 4H); 3.38 (m, 1H); 4.15 (q, 2H, $CO_2CH_2CH_3$); 5.45 (2d, 2H, Ar $CH_2$); 6.78-7.40 (m, 7H, Ar).

Example 15

Ethyl 9-p-chlorobenzyl-5,7-dichloro-1,2,3,4-tetrahydrocarbazol-1-yl-acetate

Following the procedure of Example 1, but using 1-(4-chlorobenzyl)-1-(3,5-dichlorophenyl)-hydrazine hydrochloride and ethyl 2-cyclohexanone acetate as starting materials, the title compound is prepared.

Example 16

Ethyl 9-p-chlorobenzyl-6,8-dichloro-1,2,3,4-tetrahydrocarbazol-1-yl-acetate

Following the procedure of Example 1, but using 1-(4-chlorobenzyl)-1-(2,4-dichlorophenyl)-hydrazine hydrochloride and ethyl 2-cyclohexanone acetate as starting materials, the title compound is prepared.

Example 17

(-)9-p-chlorobenzyl-6-fluoro-1,2,3,4-tetrahydrocarbazol-1-yl-acetic acid, methyl ester Step I

To 1.59 g of ethyl ester from Example 1, in 10 cc of methanol was added 10 cc of water and 420 mg of potassium hydroxide. The resulting solution was refluxed for 4 hours. Upon cooling the reaction mixture was

then acidified with HCl (1N). The resulting precipitate was filtered and washed with water. Analytically pure material was prepared by triturating the solid with a mixture of hexane/ethyl acetate (9:1) followed by filtration and drying on a high vacuum pump to give 1.24 g of the racemic acid. (89%).

| Analysis calculated for $C_{21}H_{19}NClFO_2$ | | | | | |
|---|---|---|---|---|---|
| | C | H | N | Cl | F |
| Calculated | 67.83 | 5.15 | 3.77 | 9.53 | 5.11 |
| Found | 67.88 | 5.47 | 3.63 | 9.52 | 5.12 |

Step II

10.0 g of 9-p-chlorobenzyl-6-fluoro-1,2,3,4-tetrahydrocarbazol-1-yl-acetic acid from Step I was dissolved in a mixture of hot (refluxing) acetonitrile (150 cc) and ethanol (25cc), and 4.4 g of d(+) ephedrine was added. The reflux was continued for 15 minutes and the hot solution was filtered and allowed to cool to room temperature. Crystals separated from the solution and were separated by filtration. After three recrystallizations form acetonitrile 3.9 g of the pure salt was obtained.

Step III

3.9 g of pure salt from Step II was dissolved in 200 cc of methanol and acidified using 1N hydrochloric acid. Water was added and the crystals were separated by filtration and dryed under vaccum. Upon trituration with hexane-ethyl acetate (9:1) the resolved acid was prepared.

$\alpha D$ = -42.5 (methanol) m.p. 151 - 151.5°C.

Step IV

To a solution of 1.0 g of the acid from Step III in 50 ml of ether is added a solution of diazomethane in ether until a slight excess of diazomethane is present. The excess diazomethane is destroyed by addition of a few drops of acetic acid. The reaction mixture is washed with 50 ml of 5% $Na_2CO_3$ solution, water, and dried over $MgSO_4$. Filtration and evaporation of the solvent leaves the title compound.

Example 18

(+) 9-p-chlorobenzyl-6-fluoro-1,2,3,4-tetrahydrocarbazol-1-yl-acetic acid, methyl ester

Following the method of Example 17, but using 1(-) ephedrine in Step II, there is obtained the title compound.

Example 19

Ethyl 9-p-chlorobenzyl-6-trifluoromethyl-1,2,3,4-tetrahydrocarbazol-1-yl-acetate

Following the procedure of Example 1, but using 1-(4-chlorobenzyl)-1-(4-trifluoromethylphenyl) hydrazine hydrochloride and ethyl 2-cyclohexanone acetate as starting materials, the title compound was prepared as a mixture of ethyl and isopropyl esters.

Example 20

Ethyl 9-p-chlorobenzyl-6-methylthio-1,2,3,4-tetrahydrocarbazol-1-yl-acetate

Following the procedure of Example 1, but using 1-(4-chlorobenzyl)-1-(4-methylthiophenyl) hydrazine hydrochloride and ethyl 2-cyclohexanone acetate as starting materials, the title compound was prepared as a mixture of ethyl and isopropyl esters. The pure title compound was obtained by purification on a flash chromatogram.

Example 21

Ethyl 9-p-chlorobenzyl-6-methylsulfinyl-1,2,3,4-tetrahydrocarbazol-1-yl-acetate

To 498 mg of ethyl 9-p-chlorobenzyl-6-methylthio-1,2,3,4-tetrahydrocarbazol-1-yl-acetate from Example 20 in 10 cc of methylene chloride was added 300 mg of m-chloro perbenzoic acid. The resulting mixture was stirred for 1.5 hours at room temperature. The reaction mixture was diluted with ether and consecutively washed with a solution of sodium bicarbonate, water and brine. The crude product obtained after evaporation of the organic layer was purified on silica-gel by flash chromatography eluting with 20% hexane/ethyl acetate and yielded 420 mg (82%) of the pure title compound.

Example 22

Ethyl 9-p-chlorobenzyl-6-methylsulfonyl-1,2,3,4-tetrahydrocarbazol-1-yl-acetate

To 439 mg of ethyl 9-p-chlorobenzyl-6-methylsulfinyl-1,2,3,4-tetrahydrocarbazol-1-yl-acetate from Example 21 , in 10 cc of methylene chloride was added 353 mg of m-chloro perbenzoic acid. The resulting mixture was stirred for 18 hours at room temperature. The reaction mixture was diluted with ether and washed consecutively with a solution of sodium bicarbonate, water and brine. The crude product obtained after evaporation of the organic layer was purified on silica gel by flash chromatography eluting with 30% hexane/ethyl acetate and yielded 200 mg (42%) of the pure title compound.

Example 23

Ethyl 9-p-chlorobenzyl-8-isopropyl-1,2,3,4-tetrahydrocarbazol-1-yl-acetate

Following the procedure of Example 1, but using 1-(4-chlorobenzyl)-1-(2-isopropylphenyl) hydrazine hydrochloride and ethyl 2-cyclohexanone acetate as starting materials, the title compound was prepared as a mixture of ethyl and isopropyl esters.

Example 24

9-p-Chlorobenzyl-8-methylthio-1,2,3,4-tetrahydrocarbazol-1-yl-acetic acid, methyl ester

Following the procedure of Example 27, but using 1-(2-methylthiophenyl) hydrazine hydrochloride and ethyl 2-cyclohexanone acetate as starting materials, the title compound was prepared.

Example 25

9-p-Chlorobenzyl-8-methylsulfinyl-1,2,3,4-tetrahydrocarbazol-1-yl-acetic acid, methyl ester

Following the procedure of Example 21, but using methyl ester from Example 24, there is obtained the title compound.

Example 26

Ethyl 9-p-chlorobenzyl-6-fluoro-3-methyl-1,2,3,4-tetrahydrocarbazol-1-yl-acetate

Following the procedure of Example 1, but using 1-(4-chlorobenzyl)-1-(4-fluorophenyl) hydrazine hydrochloride and ethyl 4-methyl-2-cyclohexanone acetate as starting materials, the title compound was prepared as a mixture of ethyl and isopropyl esters.

Example 27

9-p-Chlorobenzyl-6,8-difluoro-1,2,3,4-tetrahydrocarbazol-1-yl-acetic acid, methyl ester

Step I

To 114 g of 1-(2,4-difluorophenyl) hydrazine hydrochloride in 350 cc of 2-propanol containing 40 cc of acetyl chloride was added 138 g of ethyl 2-cyclohexanone acetate. The reaction was refluxed under nitrogen for 2 days. After cooling, 200 cc of ether was added and the precipitate filtered. The filtrate was evaporated to dryness. The resulting residue was dissolved in a (1:1) mixture of ether/ethyl acetate and consecutively ashed with water, sodium bicarbonate solutin and brine. The organic layer was dried over sodium sulfate and evaporated to dryness. The crude product was passed through a silica gel bed eluting with 5% ethyl acetate/hexane to yield 84 g of a 1:2 mixture of ethyl and isopropyl esters.

Step II

84 g of esters from Step I was dissolved in 250 cc of methanol and 400 cc of sodium hydroxide (1N) was added and refluxed 4 hours. After cooling, the reaction mixture was washed with a (1:1) mixture of ether/hexane and the aqueous layer was acidified with HCl (1N). The resulting precipate was filtered, washed with water and air dried to afford 50 g of 6,8-difluoro-1,2,3,4-tetrahydrocarbazol-1-yl-acetic acid.

Step III

A solution of 11.1 g of acid from Step II in 100 cc of THF was added portionwise 10.3 g of potassium tert-butoxide. The resulting mixture was stirred for 45 min at room temperature and 10.3 g p-chlorobenzyl bromide was added portionwise. The reaction mixture was stirred 18 hours at room temperature. The resulting mixture was diluted with 100 cc of water and washed with hexane. The aqueous layer was acidified with HCl (1N) and the resulting precipitate filtered, washed with water and air-dried to afford 9.4 g of 9-p-chlorobenzyl-6,8-difluoro1,2,3,4-tetrahydrocarbazol-1-yl-acetic acid.

Step IV

Following the method of Example 17, Step IV but using the acid of Step III, there was obtained the title compound.
$^1H$ NMR ($CDCl_3$) $\delta$ 1.75-2.00 (m, 4H, $(CH_2)_2$) 2.38-2.78 (m, 4H, $CH_2CO_2Me$, $CH_2$-C=C), 3.35-3.45 (m, 1H, CH-C=C), 3.70 (s, 3H, $CO_2Me$), 5.40 (dd, 1H, $\underline{CH_2}$-Ar), 6.60 (ddd, 1H, $H_7$), 6.92 (dd, 1H, $H_5$), 6.82 and 7.22 (2d, 4H, Ar).

Example 28

9-p-Chlorobenzyl-6-methoxy-8-methyl-1,2,3,4-tetrahydrocarbazol-1-yl-acetic acid, methyl ester

Following the procedure of Example 27, but using 1-(4-methoxy-2-methylphenyl) hydrazine hydrochloride and ethyl 2-cyclohexanone acetate in Step I, as starting material, the title compound is prepared.

Example 29

(-) 9-p-Chlorobenzyl-6,8-difluoro-1,2,3,4-tetrahydrocarbazol-1-yl-acetic acid, methyl ester

Following the method of Example 17, Step II to Step IV, but using 9-p-chlorobenzyl-6,8-difluoro-1,2,3,4-tetrahydrocarbazol-1-yl-acetic acid from Example 27, Step III and using d(+)ephedrine in Step III, there was obtained the title compound.
$^1H$ NMR ($CDCl_3$) $\delta$ 1.75-2.00 (m, 4H, $(CH_2)_2$) 2.38-2.78 (m, 4H, $CH_2CO_2Me$, $CH_2$-C=C), 3.35-3.45 (m, 1H, CH-C=C), 3.70 (s, 3H, $CO_2Me$), 5.40 (dd, 1H, $\underline{CH_2}$Ar), 6.60 (ddd, 1H, $4_7$), 6.92 (dd, 1H, $H_5$), 6.82 and 7.22 (2d, 4H, Ar).

Example 30

(+) 9-p-Chlorobenzyl-6,8-difluoro-1,2,3,4-tetrahydrocarbazol-1-yl-acetic acid, methyl ester

Following the method of Example 17, Step II to Step IV, but using 9-p-chlorobenzyl-6,8-difluoro1,2,3,4-tetrahydrocarbazol-1-yl-acetic acid from Example 27, Step III and using 1(-)ephedrine in Step III, there is obtained the title compound.

Example 31

(-) 9-p-Chlorobenzyl-8-methyl-1,2,3,4-tetrahydrocarbazol-1-yl-acetic acid, methyl ester

Following the method of Example 17, but using the ethyl ester from example 11 in Step I and using d-(+)ephedrine in Step II, there is obtained the title compound.

Example 32

(+) 9-p-Chlorobenzyl-8-methyl-1,2,3,4-tetrahydrocarbazol-1-yl-acetic acid, methyl ester

Following the method of Example 17, but using ethyl ester from Example 11 in Step I and using 1(-)ephedrine in Step II, there is obtained the title compound.

Example 33

(-) 9-p-Chlorobenzyl-8-fluoro-1,2,3,4-tetrahydrocarbazol-1-yl-acetic acid, methyl ester

Following the method of Example 17, but using the ethyl ester from Example 15 in Step I and using d-(+)ephedrine in Step II, there is obtained the title compound.

Example 34

(+) 9-p-Chlorobenzyl-8-fluorol-1,2,3,4-tetrahydrocarbazol-1-yl-acetic acid, methyl ester

Following the method of Example 17, but using the ethyl ester from Example 15 in Step I and using 1(-)ephedrine in Step II, there is obtained the title compound.

**Claims**

**1.** A pharmaceutical composition for inhibiting leukotriene synthesis and antagonizing prostaglandins in mammals comprising an effective amount of a compound of the formula:

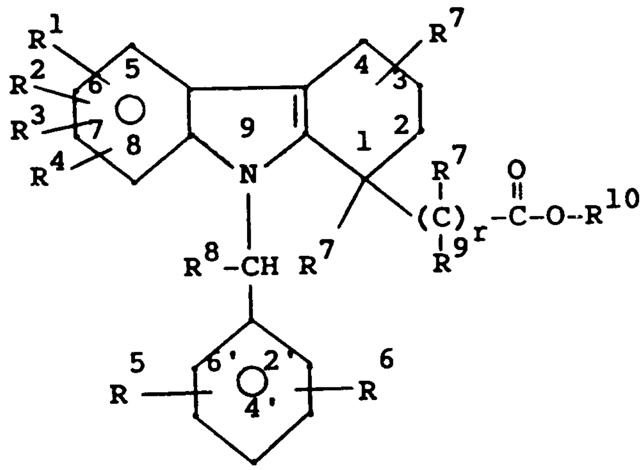

I

wherein:

$R^1$, $R^2$, $R^3$, $R^4$, $R^5$ and $R^6$ are each independently selected from:

(1) hydrogen;
(2) alkyl having 1 to 6 carbons;
(3) alkenyl having 2 to 6 carbons;
(4) $-(CH_2)_nM$
wherein n is 0 to 3 and M is

a) $OR^{11}$;
b) halogen;
c) $CF_3$;
d) $SR^{11}$;
e) phenyl or substituted phenyl;
f) $COOR^{12}$;
g)

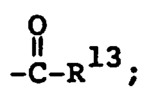

h) tetrazole;
i)

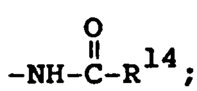

j) $-NR^{12}R^{12}$;
k) $-NHSO_2R^{15}$;
l)

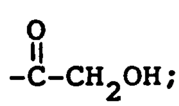

m) $-SOR^{11}$;
n) $-CONR^{12}R^{12}$;
o) $-SO_2NR^{12}R^{12}$;
p) $-SO_2R^{11}$;

q) $NO_2$;

r)

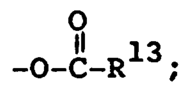

s)

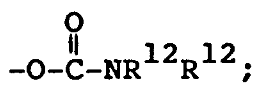

t)

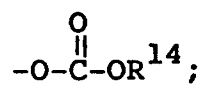

u) CN;

v) $N_3$;

$R^7$ is H or alkyl of 1 to 6 carbons;

$R^8$ is H or alkyl of 1 to 6 carbons;

each $R^9$ is independently H, OH, $C_1$ to $C_4$-O-alkyl or alkyl of 1 to 4 carbons;

$R^{10}$ is $C_1$-$C_6$ alkyl, substituted or unsubstituted 2-phenethyl, substituted or unsubstituted benzyl, substituted or unsubstituted phenyl, or

$-(CH_2)_t-C(R^7)_2-(CH_2)_t-R^{16}$

each $R^{11}$ is independently H; $C_1$ to $C_6$ alkyl; benzyl; phenyl or substituted phenyl;

each $R^{12}$ is independently H, phenyl, benzyl or $C_1$ to $C_6$ alkyl;

each $R^{13}$ is independently H, $(CH_2)_mCOOR^{12}$ wherein m is 0 to 4, $C_1$ to $C_6$ alkyl, $CF_3$, phenyl, or substituted phenyl;

each $R^{14}$ is independently $C_1$ to $C_6$ alkyl, benzyl or phenyl;

each $R^{15}$ is independently $C_1$ to $C_6$ alkyl, 4-methylphenyl, phenyl, or $CF_3$;

$R^{16}$ is A) a monocyclic or bicyclic heterocyclic radical containing from 3 to 12 nuclear carbon atoms and 1 or 2 nuclear heteroatoms selected from N, S, or O and with each ring in the heterocyclic radical being formed of 5 or 6 atoms, or B) the radical W-$R^{17}$;

$R^{17}$ contains up to 21 carbon atoms and is (1) a hydrocarbon radical or (2) an acyl radical of an organic acyclic or monocyclic carboxylic acid containing not more than 1 heteroatom in the ring;

W is O, S or NH;

r is 1 to 6

t is 0 to 3;

the substituted phenyl, substituted benzyl and substituted phenethyl groups referred to above being substituted on the benzene ring by 1 or 2 substituents selected from $C_1$ to $C_3$ alkyl, halogen, CN, $CF_3$, $COOR^{12}$, $CH_2COOR^{12}$ or $C_1$ to $C_3$ alkoxy;

or a pharmaceutically acceptable salt thereof;

and a pharmaceutically acceptable carrier.

**2.** A compound of the formula:

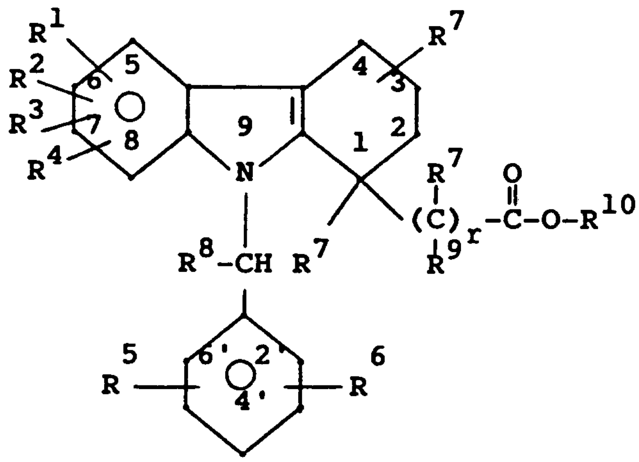

I

wherein:

$R^1$, $R^2$, $R^3$, $R^4$, $R^5$ and $R^6$ are each independently selected from:

(1) hydrogen;

(2) alkyl having 1 to 6 carbons;

(3) alkenyl having 2 to 6 carbons;

(4) $-(CH_2)_nM$

wherein n is 0 to 3 and M is

a) $OR^{11}$;

b) halogen;

c) $CF_3$;

d) $SR^{11}$;

e) phenyl or substituted phenyl;

f) $COOR^{12}$;

g)

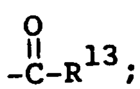

h) tetrazole;

i)

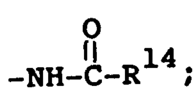

j) $-NR^{12}R^{12}$;

k) $-NHSO_2R^{15}$;

l)

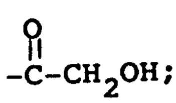

m) $-SOR^{11}$;

n) $-CONR^{12}R^{12}$;
o) $-SO_2NR^{12}R^{12}$;
p) $-SO_2R^{11}$;
q) $NO_2$;
r)

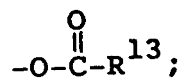

s)

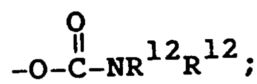

t)

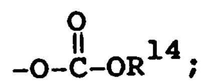

u) CN;
v) $N_3$;

provided that when $R^1$, $R^2$, $R^3$ or $R^4$ is alkyl having 1 to 6 carbons, it is not located at position 6;

$R^7$ is H or alkyl of 1 to 6 carbons;

$R^8$ is H or alkyl of 1 to 6 carbons;

each $R^9$ is independently H, OH, $C_1$ to $C_4$-O-alkyl or alkyl of 1 to 4 carbons;

$R^{10}$ is $C_1$-$C_6$ alkyl, substituted or unsubstituted 2-phenethyl, substituted or unsubstituted benzyl, substituted or unsubstituted phenyl, or

$-(CH_2)_t-C(R^7)_2-(CH_2)_t-R^{16}$

each $R^{11}$ is independently H; $C_1$ to $C_6$ alkyl; benzyl; phenyl or substituted phenyl;

each $R^{12}$ is independently H, phenyl, benzyl or $C_1$ to $C_6$ alkyl; and,

each $R^{13}$ is independently H, $(CH_2)_mCOOR^{12}$ wherein m is 0 to 4, $C_1$ to $C_6$ alkyl, $CF_3$, phenyl, or substituted phenyl;

each $R^{14}$ is $C_1$ to $C_6$ alkyl, benzyl or phenyl;

each $R^{15}$ is $C_1$ to $C_6$ alkyl, 4-methylphenyl, phenyl, or $CF_3$;

$R^{16}$ is A) a monocyclic or bicyclic heterocyclic radical containing from 3 to 12 nuclear carbon atoms and 1 or 2 nuclear heteroatoms selected from N, S, or O and with each ring in the heterocyclic radical being formed of 5 or 6 atoms, or B) the radical $W-R^{17}$;

$R^{17}$ contains up to 21 carbon atoms and is (1) a hydrocarbon radical or (2) an acyl radical of an organic acyclic or monocyclic carboxylic acid containing not more than 1 heteroatom in the ring;

W is O, S or NH;

r is 1 to 6

t is 0 to 3;

the substituted phenyl, substituted benzyl and substituted phenethyl groups referred to above being substituted on the benzene ring by 1 or 2 substituents selected from $C_1$ to $C_3$ alkyl, halogen, CN, $CF_3$, $COOR^{12}$, $CH_2COOR^{12}$ or $C_1$ to $C_3$ alkoxy;

or a pharmaceutically acceptable salt thereof.

**3.** A compound according to Claim 2 which is: ethyl 9-p-chlorobenzyl-6-fluoro-1,2,3,4-tetrahydrocarbazol-1-yl-acetate;

methyl 3-(9-p-chlorobenzyl-6-fluoro-1,2,3,4-tetrahydrocarbazol-1-yl)-propanoate;

methyl 3-(9-p-chlorobenzyl-6-methoxy-1,2,3,4-tetrahydrocarbazol-1-yl)-propanoate;

ethyl 9-p-chlorobenzyl-6-methoxy-1,2,3,4-tetrahydrocarbazol-1-yl-acetate;

ethyl 9-benzyl-6-fluoro-1,2,3,4-tetrahydrocarbazol-1-yl-acetate;
ethyl 9-p-methoxybenzyl-6-fluoro-1,2,3,4-tetrahydrocarbazol-1-yl-acetate;
ethyl 9-(3,4-dichloro)benzyl-6-fluoro-1,2,3,4-tetrahydrocarbazol-1-yl-acetate;
ethyl 9-[1-(1-phenyl)ethyl]-6-fluoro-1,2,3,4-tetrahydrocarbazol-1-yl-acetate;
ethyl 9-p-chlorobenzyl-1,2,3,4-tetrahydrocarbazol-1-yl-acetate;
ethyl 9-p-chlorobenzyl-6-chloro-1,2,3,4-tetrahydrocarbazol-1-yl-acetate;
ethyl 9-p-chlorobenzyl-8-methyl-1,2,3,4-tetrahydrocarbazol-1-yl-acetate;
ethyl 6-bromo-9-p-chlorobenzyl-1,2,3,4-tetrahydrocarbazol-1-yl-acetate;
methyl 2-(9-p-chlorobenzyl-6-fluoro-1,2,3,4-tetrahydrocarbazol-1-yl)-propanoate;
ethyl 9-p-chlorobenzyl-8-fluoro-1,2,3,4-tetrahydrocarbazol-1-yl-acetate;
ethyl 9-p-chlorobenzyl-5,7-dichloro-1,2,3,4-tetrahydrocarbazol-1-yl-acetate;
ethyl 9-p-chlorobenzyl-6,8-dichloro-1,2,3,4-tetrahydrocarbazol-1-yl-acetate;
ethyl 9-p-chlorobenzyl-6-isopropyl-1-2,3,4-tetrahydrocarbazol-1-yl-acetate;
ethyl 9-p-chlorobenzyl-6-tert-butyl-1-2,3,4-tetrahydrocarbazol-1-yl-acetate;
ethyl 9-p-chlorobenzyl-6-trifluoromethyl-1,2,3,4-tetrahydrocarbazol-1-yl-acetate;
ethyl 9-p-chlorobenzyl-6-methylthio-1,2,3,4-tetrahydrocarbazol-1-yl-acetate;
ethyl 9-p-chlorobenzyl-6-methylsulfinyl-1,2,3,4-tetrahydrocarbazol-1-yl-acetate;
ethyl 9-p-chlorobenzyl-6-methylsulfonyl-1,2,3,4-tetrahydrocarbazol-1-yl-acetate;
ethyl 9-p-chlorobenzyl-8-isopropyl-1,2,3,4-tetrahydrocarbazol-1-yl-acetate;
9-p-Chorobenzyl-8-methylthio-1,2,3,4-tetrahydrocarbazol-1-yl-acetic acid, methyl ester;
9-p-Chlorobenzyl-8-methylsulfinyl-1,2,3,4-tetrahydrocarbazol-1-yl-acetic acid, methyl ester;
ethyl 9-p-chlorobenzyl-6-fluoro-3-methyl-1,2,3,4-tetrahydrocarbazol-1-yl-acetate;
9-p-Chlorobenzyl-6,8-difluoro-1,2,3,4-tetrahydrocarbazol-1-yl-acetic acid, methyl ester;
9-p-Chlorobenzyl-6,8-dimethyl-1,2,3,4-tetrahydrocarbazol-1-yl-acetic acid, methyl ester;
9-p-Chlorobenzyl-6-methoxy-8-methyl-1,2,3,4-tetrahydrocarbazol-1-yl-acetic acid, methyl ester;
(-) 9-p-Chlorobenzyl-6,8-difluro-1,2,3,4-tetrahydrocarbazol-1-yl-acetic acid, methyl ester;
(+) 9-p-Chlorobenzyl-6,8-difluoro-1,2,3,4-tetrahydrocarbazol-1-yl-acetic acid, methyl ester;
(-) 9-p-Chlorobenzyl-8-methyl-1,2,3,4-tetrahydrocarbazol-1-yl-acetic acid, methyl ester;
(+) 9-p-Chlorobenzyl-8-fluoro-1,2,3,4-tetrahydrocarbazol-1-yl-acetic acid, methyl ester;
(-) 9-p-Chlorobenzyl-8-fluoro-1,2,3,4-tetrahydrocarbazol-1-yl-acetic acid, methyl ester;
(+) 9-p-Chlorobenzyl-8-fluoro-1,2,3,4-tetrahydrocarbazol-1-yl-acetic acid, methyl ester.

**4.** A compound according to Claim 2, which is:
ethyl 9-p-chlorobenzyl-6-fluoro-1,2,3,4-tetrahydrocarbazol-1-yl-acetate;
ethyl 9-p-chlorobenzyl-8-methyl-1,2,3,4-tetrahydrocarbazol-1-yl-acetate;
ethyl 9-p-chlorobenzyl-8-fluoro-1,2,3,4-tetrahydrocarbazol-1-yl-acetate;
9-p-chlorobenzyl-6,8-difluoro-1,2,3,4-tetrahydrocarbazol-1-yl-acetic acid, methyl ester.

**5.** A compound according to Claim 2, which is (-)9-p-chlorobenzyl-6-fluoro-1,2,3,4-tetrahydrocarbazol-1-yl-acetic acid, methyl ester;
(+) 9-p-chlorobenzyl-6-fluoro-1,2,3,4-tetrahydrocarbazol-1-yl-acetic acid, methyl ester;
(-)9-p-chlorobenzyl-6-fluoro-1,2,3,4-tetrahydrocarbazol-1-yl-acetic acid, methyl ester;
(-) 9-p-Chlorobenzyl-6,8-difluoro-1,2,3,4-tetrahydrocarbazol-1-yl-acetic acid, methyl ester;
(-) 9-p-Chlorobenzyl-8-methyl-1,2,3,4-tetrahydrocarbazol-1-yl-acetic acid, methyl ester;
(-) 9-p-Chlorobenzyl-8-fluoro-1,2,3,4-tetrahydrocarbazol-1-yl-acetic acid, methyl ester.

**6.** A compound which is ethyl 9-p-chlorobenzyl-6- methyl-1,2,3,4,6-tetrahydrocarbazol-1-yl-acetate.

**7.** A compound of the formula:

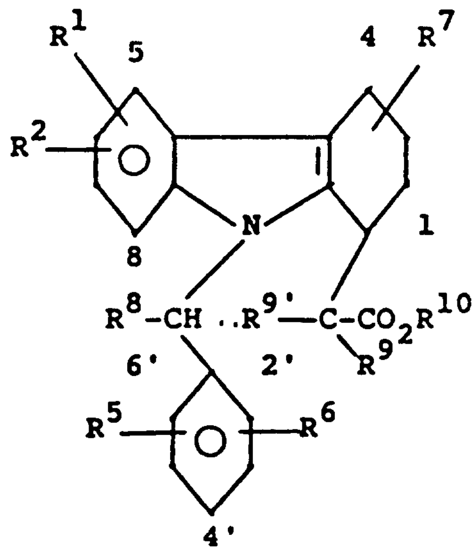

wherein:

| Compound | $R^1$ | $R^2$ | $R^5$ | $R^6$ | $R^9$, $R^{9'}$ | $R^7$ | $R^8$ | $R^{10}$ |
|---|---|---|---|---|---|---|---|---|
| 1 (Ex.1) | 6-F | H | 4'-Cl | H | H, H | H | H | Et |
| 2 (Ex. 4) | 6-OMe | H | 4'-Cl | H | H, H | H | H | Et |
| 3 (Ex. 18) | 6-F (-) isomer | H | 4'-Cl | H | H, H | H | H | Me |
| 4 (Ex. 19) | 6-F (+) isomer | H | 4'-Cl | H | H, H | H | H | Me |
| 5 (Ex. 5) | 6-F | H | H | H | H, H | H | H | Et |
| 6 (Ex. 6) | 6-F | H | 4'-OMe | H | H, H | H | H | Et |
| 7 (Ex. 7) | 6-F | H | 3'-Cl | 4'-Cl | H, H | H | H | Et |
| 8 (Ex. 8) | 6-F | H | H | H | H, H | H | Me | Et |
| 9 (Ex 9) | H | H | 4'-Cl | H | H, H | H | H | Et |
| 10 (Ex. 10) | 6-Cl | H | 4'-Cl | H | H, H | H | H | Et |
| 11 (Ex. 11) | 8-Me | H | 4'-Cl | H | H, H | H | H | Et |
| 12 (Ex. 12) | 6-Br | H | 4'-Cl | H | H, H | H | H | Et |

| Compound | $R^1$ | $R^2$ | $R^5$ | $R^6$ | $R^9$, $R^{9'}$ | $R^7$ | $R^8$ | $R^{10}$ |
|---|---|---|---|---|---|---|---|---|
| 13 (Ex. 14) | 8-F | H | 4'-Cl | H | H, H | H | H | Et |
| 14 | 6-F | H | 4'-Cl | H | H, H | 3-t-Bu | H | $CH_2C_6H_5$ |
| 15 | 5-F | H | 4'-Cl | H | H, H | H | H | $CH_2OAc$ |
| 16 | 7-F | H | 4'-Cl | H | H, H | H | H | 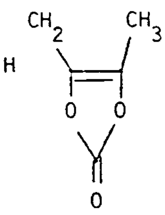 |
| 17 (Ex. 15) | 5-Cl | 7-Cl | 4'-Cl | H | H, H | H | H | Et |
| 18 (Ex. 16) | 6-Cl | 8-Cl | 4'-Cl | H | H, H | H | H | Et |
| 19 | 6-F | H | 4'-Cl | H | Me, H | H | H | 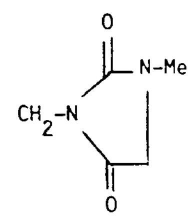 |
| 20 | 6-F | H | 4'-Cl | H | Me, Me | H | H | 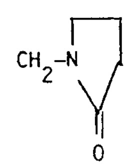 |
| 21 | 6-F | H | 4'-Cl | H | H, H | 1-Me | H | 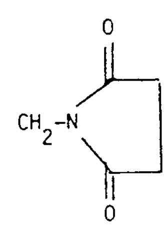 |

| Compound | $R^1$ | $R^2$ | $R^5$ | $R^6$ | $R^9,R^{9'}$ | $R^7$ | $R^8$ | $R^{10}$ |
|---|---|---|---|---|---|---|---|---|
| 22 (ex 19) | 6-$CF_3$ | H | 4'-Cl | H | H,H | H | H | Et |
| 23 (ex 20) | 6-SMe | H | 4'-Cl | H | H,H | H | H | Et |
| 24 (ex 21) | 6-SOMe | H | 4'-Cl | H | H,H | H | H | Et |
| 25 (ex 22) | 6-$SO_2$Me | H | 4'-Cl | H | H,H | H | H | Et |
| 26 (ex 23) | 8-$CH(Me)_2$ | H | 4'-Cl | H | H,H | H | H | Et |
| 27 (ex 24) | 8-SMe | H | 4'-Cl | H | H,H | H | H | Me |
| 28 (ex 25) | 8-SOMe | H | 4'-Cl | H | H,H | H | H | Me |
| 29 (ex 26 | 6-F | H | 4'-Cl | H | H,H | 3-Me | H | Et |
| 30 (ex 27) | 6-F | 8-F | 4'-Cl | H | H,H | H | H | Me |
| 31 (ex 28 | 6-OMe | 8-Me | 4'-Cl | H | H,H | H | H | Me |
| 32 (ex 29) | 6-F(-)Isomer | 8-F | 4'-Cl | H | H,H | H | H | Me |
| 33 (ex 30) | 6-F(+)Isomer | 8-F | 4'-Cl | H | H,H | H | H | Me |
| 34 (ex 31) | 8-Me(-)Isomer | H | 4'-Cl | H | H,H | H | H | Me |
| 35 (ex 32) | 8-Me(+)Isomer | H | 4'-Cl | H | H,H | H | H | Me |
| 36 (ex 33) | 8-F(-)Isomer | H | 4'-Cl | H | H,H | H | H | Me |
| 37 (ex 34 | 8-F(+)Isomer | H | 4'-Cl | H | H,H | H | H | Me |
| 38 | 6-F | 8-F | 3'-Cl | 4'-Cl | H,H | H | H | Me |
| 39 | 6-F | 8-F | 2'-Cl | 4'-Cl | H,H | H | H | Me |
| 40 | 6-F | 8-F | 4'-OMe | H | H,H | H | H | Me |
| 41 | 6-F | 8-F | 4'-OH | H | H,H | H | H | Me |
| 42 | 6-F | 8-F | 4'-SMe | H | H,H | H | H | Me |
| 43 | 6-F | H | 4'-S(O)Me | H | H,H | H | H | Me |
| 44 | 6-F | 8-F | 4'-NHCOMe | H | H,H | H | H | Me |
| 45 | 6-F | H | 4'-$S(O)_2$Me | H | H,H | H | H | Me |

| Compound | $R^1$ | $R^2$ | $R^5$ | $R^6$ | $R^9,R^{9'}$ | $R^7$ | $R^8$ | $R^{10}$ |
|---|---|---|---|---|---|---|---|---|
| 46 | 6-F | H | 4'-F | H | H,H | H | H | Me |
| 47 | 6-F | H | 4'-Br | H | H,H | H | H | Me |
| 48 | 6-F | 8-Me | 4'-Cl | H | H,H | H | H | Me |
| 49 | 6-F | H | 4'-$CO_2$H | H | H,H | H | H | Me |
| 50 | 6-F | H | 4'-$CO_2$Me | H | H,H | H | H | Me |
| 51 | 6-F | 8-F | 4'-n-$C_3H_7$ | H | H,H | H | H | Me |
| 52 | 6-F | 8-F | 3'-I | 4'-OH | H,H | H | H | Me |
| 53 | 6-F | 8-F | 4'-I | H | H,H | H | H | Me |
| 54 | 6-$N_3$ | H | 4'-Cl | H | H,H | H | H | Me |
| 55 | 6-F | H | 4'-$N_3$ | H | H,H | H | H | Me |

**8.** The use of a compound of formula (I) as defined in Claim 1 for the manufacture of a medicament useful for either inhibiting leukotriene synthesis or antagonizing prostaglandins in mammals.

**9.** A pharmaceutical composition which comprises a composition of Claim 1 and an effective amount of a second active ingredient selected from the group consisting of non-steroidal anti-inflammatory drugs; peripheral analgesic agents; cyclooxygenase inhibitors; leukotriene antagonists; leukotriene biosynthesis inhibitors; $H_1$-receptor antagonists; $H_2$ receptor antagonists; prostaglandin antagonists; ACE inhibitors, thromboxane synthetase inhibitors, or serotonin antagonists.

**10.** The use of a compound as claimed in Claim 2 for the manufacture of a medicament useful for either inhibiting leukotriene synthesis or antagonizing prostaglandins in mammals.

**Patentansprüche**

**1.** Pharmazeutisches Präparat zur Hemmung der Leukotriensynthese und Antagonisierung von Prostaglandinen bei Säugern, umfassend eine wirksame Menge einer Verbindung der Formel:

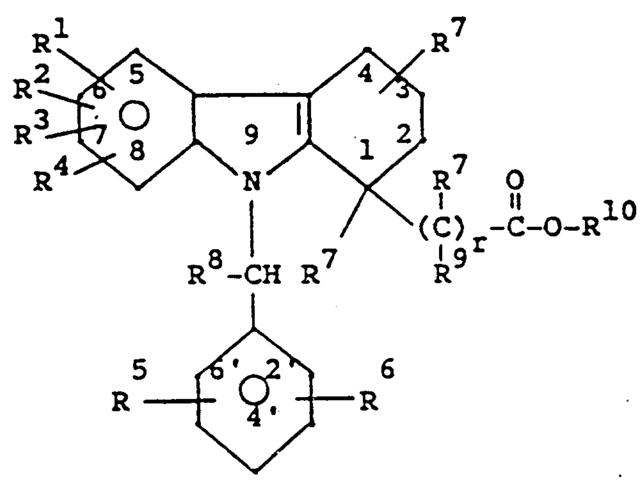

worin:

$R^1$, $R^2$, $R^3$, $R^4$, $R^5$ und $R^6$ jeweils unabhängig ausgewählt sind aus:

(1) Wasserstoff;

(2) Alkyl mit 1 bis 6 Kohlenstoffen;

(3) Alkenyl mit 2 bis 6 Kohlenstoffen;

(4) $-(CH_2)_nM$

worin n eine Zahl von 0 bis 3 bedeutet und M

a) $OR^{11}$;

b) Halogen;

c) $CF_3$;

d) $SR^{11}$;

e) Phenyl oder substituiertes Phenyl;

f) $COOR^{12}$;

g)

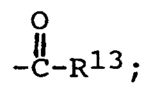

h) Tetrazol;

i)

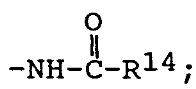

j) $-NR^{12}R^{12}$;

k) $-NHSO_2R^{15}$;

l)

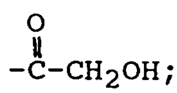

m) $-SOR^{11}$;

n) $-CONR^{12}R^{12}$;

o) $-SO_2NR^{12}R^{12}$;

p) $-SO_2R^{11}$;

q) $NO_2$;

r)

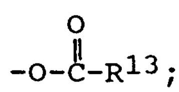

s)

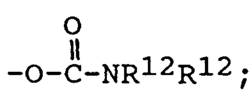

t)

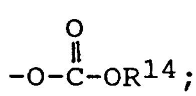

u) CN;

v) $N_3$
darstellt;
$R^7$ H oder Alkyl mit 1 bis 6 Kohlenstoffen bedeutet;
$R^8$ für H oder Alkyl mit 1 bis 6 Kohlenstoffen steht;
$R^9$ jeweils unabhängig H, OH, $C_1$- bis $C_4$-O-Alkyl oder Alkyl mit 1 bis 4 Kohlenstoffen bedeutet;
$R^{10}$ $C_1$- bis $C_6$-Alkyl, substituiertes oder unsubstituiertes 2-Phenethyl, substituiertes oder unsubstituiertes Benzyl substituiertes oder unsubstituiertes Phenyl oder $-(CH_2)_t-C(R^7)_2-(CH_2)_t-R^{16}$ darstellt;
$R^{11}$ jeweils unabhängig für H, $C_1$- bis $C_6$-Alkyl, Benzyl, Phenyl oder substituiertes Phenyl steht;
$R^{12}$ jeweils unabhängig H, Phenyl, Benzyl oder $C_1$- bis $C_6$-Alkyl bedeutet;
$R^{13}$ jeweils unabhängig H, $(CH_2)_mCOOR^{12}$, worin m eine Zahl von 0 bis 4 ist, $C_1$- bis $C_6$-Alkyl, $CF_3$, Phenyl oder substituiertes Phenyl bedeutet;
$R^{14}$ jeweils unabhängig für $C_1$- bis $C_6$-Alkyl, Benzyl oder Phenyl steht;
$R^{15}$ jeweils unabhängig $C_1$- bis $C_6$-Alkyl, 4-Methylphenyl, Phenyl oder $CF_3$ bedeutet;
$R^{16}$ A) ein monocyclisches oder bicyclisches, heterocyclisches Radikal darstellt, das im Kern 3 bis 12 Kohlenstoffatome und 1 bis 2 Heteroatome enthält, die aus H, S oder O ausgewählt sind, und wobei der Ring in dem heterocyclischen Radikal jeweils aus 5 oder 6 Atomen gebildet ist, oder B) das Radikal W-$R^{17}$ darstellt;
$R^{17}$ bis zu 21 Kohlenstoffatome enthält und (1) ein Kohlenwasserstoffradikal oder (2) ein Acylradikal einer organischen acyclischen oder monocylischen Carbonsäure darstellt, die nicht mehr als ein Heteroatom im Ring enthält;
W O, S oder NH bedeutet;
R eine Zahl von 1 bis 6 ist;
t eine Zahl von 0 bis 3 ist;
wobei die obengenannten substituierten Phenylsubstituierten Benzyl-und substituierten PhenethylGruppen am Benzolring mit 1 oder 2 Substiuenten substiuiert sind, die aus $C_1$- bis $C_3$-Alkyl, Halogen, CN, $CF_3$, $COOR^{12}$, $CH_2COOR_{12}$ oder $C_1$- bis $C_3$-Aklkoxy ausgewählt sind;
oder ein pharmazeutisch verträgliches Salz davon und ein pharmazeutisch verträglicher Trägerstoff.

**2.** Verbindung der Formel:

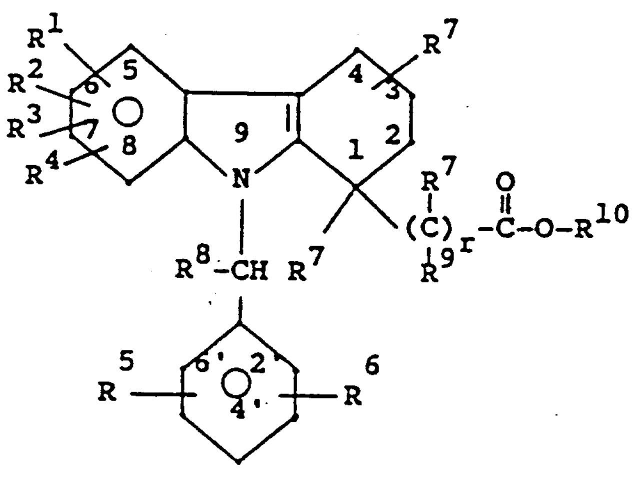

worin:
$R^1$, $R^2$, $R^3$, $R^4$, $R^5$ und $R^6$ jeweils unabhängig ausgewählt sind aus:
(1) Wasserstoff;
(2) Alkyl mit 1 bis 6 Kohlenstoffen;
(3) Alkenyl mit 2 bis 6 Kohlenstoffen;
(4) $-(CH_2)_nM$
worin n eine Zahl von 0 bis 3 bedeutet und M
a) $OR^{11}$;
b) Halogen;

c) $CF_3$;
d) $SR^{11}$;
e) Phenyl oder substituiertes Phenyl;
f) $COOR^{12}$;
g)

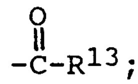

h) Tetrazol;
i)

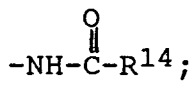

j) $-NR^{12}R^{12}$;
k) $-NHSO_2R^{15}$;
l)

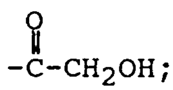

m) $-SOR^{11}$;
n) $-CONR^{12}R^{12}$;
o) $-SO_2NR^{12}R^{12}$;
p) $-SO_2R^{11}$;
q) $NO_2$;
r)

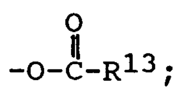

s)

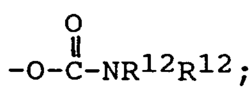

t)

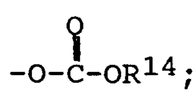

u) CN;
v) $N_3$;
bedeutet,

mit der Maßgabe, daß, wenn $R^1$, $R^2$, $R^3$ oder $R^4$ Alkyl mit 1 bis 6 Kohlenstoffatomen bedeutet, es nicht an Position 6 gebunden ist;

$R^7$ H oder Alkyl mit 1 bis 6 Kohlenstoffen darstellt;
$R^8$ für H oder Alkyl mit 1 bis 6 Kohlenstoffen steht;
$R^9$ jeweils unabhängig H, OH, $C_1$- bis $C_4$-O-Alkyl oder Alkyl mit 1 bis 4 Kohlenstoffen bedeutet;
$R^{10}$ $C_1$- bis $C_6$-Alkyl, substituiertes oder unsubstituiertes 2-Phenethyl, substituiertes oder unsubstituiertes Benzyl, substituiertes oder unsubstituiertes Phenyl oder

$-(CH_2)_t-C(R^7)_2-(CH_2)_tR^{16}$ bedeutet;

$R^{11}$ jeweils unabhängig für H, $C_1$- bis $C_6$-Alkyl, Benzyl, Phenyl oder substituiertes Phenyl steht;

$R^{12}$ jeweils unabhängig H, Phenyl, Benzyl oder $C_1$- bis $C_6$-Alkyl bedeutet;

$R^{13}$ jeweils unabhängig H, $(CH_2)_mCOOR^{12}$, worin m eine Zahl von 0 bis 4 ist, $C_1$- bis $C_6$-Alkyl, $CF_3$, Phenyl oder substituiertes Phenyl bedeutet;

$R^{14}$ jeweils $C_1$- bis $C_6$-Alkyl, Benzyl oder Phenyl darstellt;

$R^{15}$ jeweils $C_1$- bis $C_6$-Alkyl, 4-Methylphenyl, Phenyl oder $CF_3$ darstellt;

$R^{16}$ A) für ein monocyclisches oder bicyclisches, heterocyclisches Radikal steht, das im Kern 3 bis 12 Kohlenstoffatome und 1 bis 2 Heteroatome enthält, die aus N, S oder O ausgewählt sind, und wobei der Ring in dem heterocyclischen Radikal jeweils aus 5 oder 6 Kohlenstoffatomen gebildet ist, oder B) für das Radikal $W-R^{17}$ steht;

$R^{17}$ bis zu 21 Kohlenstoffatome enthält und (1) ein Kohlenwasserstoffradikal oder (2) ein Acylradikal einer organischen, acyclischen oder monocylischen Carbonsäure darstellt, die nicht mehr als ein Heteroatom im Ring enthält;

W O, S oder NH darstellt;

R eine Zahl von 1 bis 6 ist;

t eine Zahl von 0 bis 3 ist;

wobei die obengenannten substituierten Phenylsubstituierten Benzyl- und substituierten Phenethylgruppen am Benzolring mit 1 oder 2 Substiuenten substiuiert sind, die aus $C_1$- bis $C_3$-Alkyl, Halogen, CN, $CF_3$, $COOR^{12}$, $CH_2COOR_{12}$ oder $C_1$-bis $C_3$-Aklkoxy ausgewählt sind;

oder ein pharmazeutisch verträgliches Salz davon.

**3.** Verbindung nach Anspruch 2, die die folgenden Verbindungen darstellt:

Ethyl-9-p-chlorbenzyl-6-fluor-1,2,3,4-tetrahydrocarbazol-1-yl-acetat;
Methyl-3-(9-p-chlorbenzyl-6-fluor-1,2,3,4-tetrahydrocarbazol-1-yl)-propanoat;
Methyl-3-(9-p-chlorbenzyl-6-methoxy-1,2,3,4-tetrahydrocarbazol-1-yl)-propanoat;
Ethyl-9-p-chlorbenzyl-6-methoxy-1,2,3,4-tetrahydrocarbazol-1-yl-acetat;
Ethyl-9-benzyl-6-fluor-1,2,3,4-tetrahydrocarbazol-1-yl-acetat;
Ethyl-9-p-methoxybenzyl-6-fluor-1,2,3,4-tetrahydrocarbazol-1-yl-acetat;
Ethyl-9-(3,4-dichlor)-benzyl-6-fluor-1,2,3,4-tetrahydrocarbazol-1-yl-acetat;
Ethyl-9-[1-(1-phenyl)-ethyl]-6-fluor-1,2,3,4-tetrahydrocarbazol-1-yl-acetat;
Ethyl-9-p-chlorbenzyl-1,2,3,4-tetrahydrocarbazol-1-yl-acetat;
Ethyl-9-p-chlorbenzyl-6-chlor-1,2,3,4-tetrahydrocarbazol-1-yl-acetat;
Ethyl-9-p-chlorbenzyl-8-methyl-1,2,3,4-tetrahydrocarbazol-1-yl-acetat;
Ethyl-6-brom-9-p-chlorbenzyl-1,2,3,4-tetrahydrocarbazol-1-yl-acetat;
Methyl-2-(9-p-chlorbenzyl-6-fluor-1,2,3,4-tetrahydrocarbazol-1-yl)-propanoat;
Ethyl-9-p-chlorbenzyl-8-fluor-1,2,3,4-tetrahydrocarbazol-1-yl-acetat;
Ethyl-9-p-chlorbenzyl-5,7-dichlor-1,2,3,4-tetrahydrocarbazol-1-yl-acetat;
Ethyl-9-p-chlorbenzyl-6,8-dichlor-1,2,3,4-tetrahydrocarbazol-1-yl-acetat;
Ethyl-9-p-chlorbenzyl-6-isopropyl-1,2,3,4-tetrahydrocarbazol-1-yl-acetat;
Ethyl-9-p-chlorbenzyl-6-tert-butyl-1,2,3,4-tetrahydrocarbazol-1-yl-acetat;
Ethyl-9-p-chlorbenzyl-6-trifluormethyl-1,2,3,4-tetrahydrocarbazol-1-yl-acetat;
Ethyl-9-p-chlorbenzyl-6-methylthio-1,2,3,4-tetrahydrocarbazol-1-yl-acetat;
Ethyl-9-p-chlorbenzyl-6-methylsulfinyl-1,2,3,4-tetrahydrocarbazol-1-yl-acetat;
Ethyl-9-p-chlorbenzyl-6-methylsulfonyl-1,2,3,4-tetrahydrocarbazol-1-yl-acetat;
Ethyl-9-p-chlorbenzyl-8-isopropyl-1,2,3,4-tetrahydrocarbazol-1-yl-acetat;
9-p-Chlorbenzyl-8-methylthio-1,2,3,4-tetrahydrocarbazol-1-yl-essigsäuremethylester;
9-p-Chlorbenzyl-8-methylsulfinyl-1,2,3,4-tetrahydrocarbazol-1-yl-essigsäuremethylester;
Ethyl-9-p-chlorbenzyl-6-fluor-3-methyl-1,2,3,4-tetrahydrocarbazol-1-yl-acetat;
9-p-Chlorbenzyl-6,8-difluor-1,2,3,4-tetrahydrocarbazol-1-yl-essigsäuremethylester;
9-p-Chlorbenzyl-6,8-dimethyl-1,2,3,4-tetrahydrocarbazol-1-yl-essigsäuremethylester;
9-p-Chlorbenzyl-6-methoxy-8-methyl-1,2,3,4-tetrahydrocarbazol-1-yl-essigsäuremethylester;
(-) 9-p-Chlorbenzyl-6,8-difluor-1,2,3,4-tetrahydrocarbazol-1-yl-essigsäuremethylester;
(+) 9-p-Chlorbenzyl-6,8-difluor-1,2,3,4-tetrahydrocarbazol-1-yl-essigsäuremethylester;
(-) 9-p-Chlorbenzyl-8-methyl-1,2,3,4-tetrahydrocarbazol-1-yl-essigsäuremethylester;
(+) 9-p-Chlorbenzyl-8-fluor-1,2,3,4-tetrahydrocarbazol-1-yl-essigsäuremethylester;
(-) 9-p-Chlorbenzyl-8-fluor-1,2,3,4-tetrahydrocarbazol-1-yl-essigsäuremethylester;

(+) 9-p-Chlorbenzyl-8-fluor-1,2,3,4-tetrahydrocarbazol-1-yl-essigsäuremethylester.

4. Verbindung nach Anspruch 2, die die folgenden Verbindungen darstellt:
Ethyl-9-p-chlorbenzyl-6-fluor-1,2,3,4-tetrahydrocarbazol-1-yl-acetat;
Ethyl-9-p-chlorbenzyl-8-methyl-1,2,3,4-tetrahydrocarbazol-1-yl-acetat;
Ethyl-9-p-chlorbenzyl-8-fluor-1,2,3,4-tetrahydrocarbazol-1-yl-acetat;
9-p-Chlorbenzyl-6,8-difluor-1,2,3,4-tetrahydrocarbazol-1-yl-essigsäuremethylester.

5. Verbindung nach Anspruch 2 die die folgenden Verbindungen darstellt:
(-)-9-p-Chlorbenzyl-6-fluor-1,2,3,4-tetrahydrocarbazol-1-yl-essigsäuremethylester;
(+)-9-p-Chlorbenzyl-6-fluor-1,2,3,4-tetrahydrocarbazol-1-yl-essigsäuremethylester;
(-)-9-p-Chlorbenzyl-6-fluor-1,2,3,4-tetrahydrocarbazol-1-yl-essigsäuremethylester;
(-)-9-p-Chlorbenzyl-6,8-difluor-1,2,3,4-tetrahydrocarbazol-1-yl-essigsäuremethylester;
(-)-9-p-Chlorbenzyl-8-methyl-1,2,3,4-tetrahydrocarbazol-1-yl-essigsäuremethylester;
(-)-9-p-Chlorbenzyl-8-fluor-1,2,3,4-tetrahydrocarbazol-1-yl-essigsäuremethylester.

6. Verbindung, die Ethyl-9-p-chlorbenzyl-6-methyl-1,2,3,4-tetrahydrocarbazol-1-yl-essigsäuremethylester ist.

7. Verbindung der Formel:

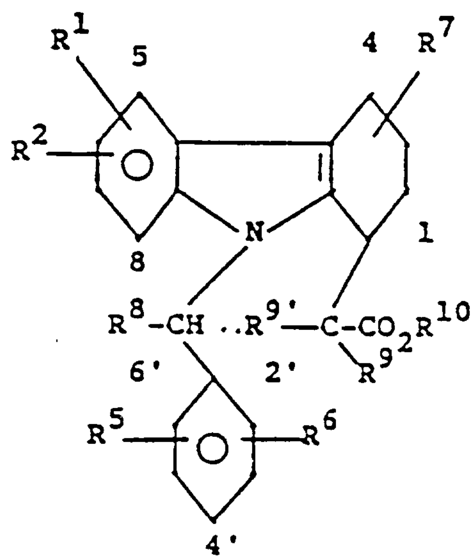

worin:

| Verbindung | | $R^1$ | $R^2$ | $R^5$ | $R^6$ | $R^9$, $R^{9'}$ | $R^7$ | $R^8$ | $R^{10}$ |
|---|---|---|---|---|---|---|---|---|---|
| 1 | Bsp. 1 | 6-F | H | 4'-Cl | H | H, H | H | H | Et |
| 2 | Bsp. 4 | 6-OMe | H | 4'-Cl | H | H, H | H | H | Et |
| 3 | Bsp. 18 | 6-F (-) isomer | H | 4'-Cl | H | H, H | H | H | Me |
| 4 | Bsp. 19 | 6-F (+) isomer | H | 4'-Cl | H | H, H | H | H | Me |
| 5 | Bsp. 5 | 6-F | H | H | H | H, H | H | H | Et |
| 6 | Bsp. 6 | 6-F | H | 4'-OMe | H | H, H | H | H | Et |
| 7 | Bsp. 7 | 6-F | H | 3'-Cl | 4'-Cl | H, H | H | H | Et |
| 8 | Bsp. 8 | 6-F | H | H | H | H, H | H | Me | Et |
| 9 | Bsp. 9 | H | H | 4'-Cl | H | H, H | H | H | Et |
| 10 | Bsp. 10 | 6-Cl | H | 4'-Cl | H | H, H | H | H | Et |
| 11 | Bsp. 11 | 8-Me | H | 4'-Cl | H | H, H | H | H | Et |
| 12 | Bsp. 12 | 6-Br | H | 4'-Cl | H | H, H | H | H | Et |

| | Verbindung | $R^1$ | $R^2$ | $R^5$ | $R^6$ | $R^9$, $R^{9'}$ | $R^7$ | $R^8$ | $R^{10}$ |
|---|---|---|---|---|---|---|---|---|---|
| 13 | Bsp. 14 | 8-F | H | 4'-Cl | H | H, H | H | H | Et |
| 14 | | 6-F | H | 4'-Cl | H | H, H | 3-t-Bu | H | $CH_2C_6H_5$ |
| 15 | | 5-F | H | 4'-Cl | H | H, H | H | H | $CH_2OAc$ |
| 16 | | 7-F | H | 4'-Cl | H | H, H | H | H | 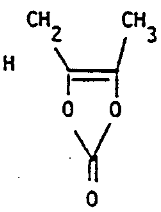 |
| 17 | Bsp. 15 | 5-Cl | 7-Cl | 4'-Cl | H | H, H | H | H | Et |
| 18 | Bsp. 16 | 6-Cl | 8-Cl | 4'-Cl | H | H, H | H | H | Et |
| 19 | | 6-F | H | 4'-Cl | H | Me, H | H | H | 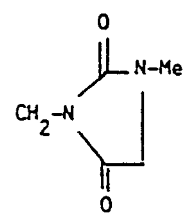 |
| 20 | | 6-F | H | 4'-Cl | H | Me, Me | H | H | 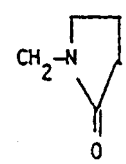 |
| 21 | | 6-F | H | 4'-Cl | H | H, H | 1-Me | H | 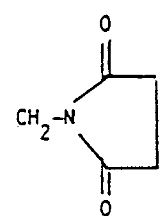 |

| Verbindung | | $R^1$ | $R^2$ | $R^5$ | $R^6$ | $R^9,R^{9'}$ | $R^7$ | $R^8$ | $R^{10}$ |
|---|---|---|---|---|---|---|---|---|---|
| 22 | Bsp. 19 | 6-$CF_3$ | H | 4'-Cl | H | H,H | H | H | Et |
| 23 | Bsp. 20 | 6-SMe | H | 4'-Cl | H | H,H | H | H | Et |
| 24 | Bsp. 21 | 6-SOMe | H | 4'-Cl | H | H,H | H | H | Et |
| 25 | Bsp. 22 | 6-$SO_2$Me | H | 4'-Cl | H | H,H | H | H | Et |
| 26 | Bsp. 23 | 8-CH$(Me)_2$ | H | 4'-Cl | H | H,H | H | H | Et |
| 27 | Bsp. 24 | 8-SMe | H | 4'-Cl | H | H,H | H | H | Me |
| 28 | Bsp. 25 | 8-SOMe | H | 4'-Cl | H | H,H | H | H | Me |
| 29 | Bsp. 26 | 6-F | H | 4'-Cl | H | H,H | 3-Me | H | Et |
| 30 | Bsp. 27 | 6-F | 8-F | 4'-Cl | H | H,H | H | H | Me |
| 31 | Bsp. 28 | 6-OMe | 8-Me | 4'-Cl | H | H,H | H | H | Me |
| 32 | Bsp. 29 | 6-F(-)-Isomeres | 8-F | 4'-Cl | H | H,H | H | H | Me |
| 33 | Bsp. 30 | 6-F(+)-Isomeres | 8-F | 4'-Cl | H | H,H | H | H | Me |
| 34 | Bsp. 31 | 8-Me(-)-Isomeres | H | 4'-Cl | H | H,H | H | H | Me |
| 35 | Bsp. 32 | 8-Me(+)-Isomeres | H | 4'-Cl | H | H,H | H | H | Me |
| 36 | Bsp.33 | 8-F(-)-Isomeres | H | 4'-Cl | H | H,H | H | H | Me |
| 37 | Bsp. 34 | 8-F(+)-Isomeres | H | 4'-Cl | H | H,H | H | H | Me |
| 38 | | 6-F | 8-F | 3'-Cl | 4'-Cl | H,H | H | H | Me |
| 39 | | 6-F | 8-F | 2'-Cl | 4'-Cl | H,H | H | H | Me |
| 40 | | 6-F | 8-F | 4'-OMe | H | H,H | H | H | Me |
| 41 | | 6-F | 8-F | 4'-OH | H | H,H | H | H | Me |
| 42 | | 6-F | 8-F | 4'-SMe | H | H,H | H | H | Me |
| 43 | | 6-F | H | 4'-S(O)Me | H | H,H | H | H | Me |
| 44 | | 6-F | 8-F | 4'-NHCOMe | H | H,H | H | H | Me |
| 45 | | 6-F | H | 4'-$S(O)_2$Me | H | H,H | H | H | Me |

| Verbindung | $R^1$ | $R^2$ | $R^5$ | $R^6$ | $R^9$,$R^{9'}$ | $R^7$ | $R^8$ | $R^{10}$ |
|---|---|---|---|---|---|---|---|---|
| 46 | 6-F | H | 4'-F | H | H,H | H | H | Me |
| 47 | 6-F | H | 4'-Br | H | H,H | H | H | Me |
| 48 | 6-F | 8-Me | 4'-Cl | H | H,H | H | H | Me |
| 49 | 6-F | H | 4'-$CO_2H$ | H | H,H | H | H | Me |
| 50 | 6-F | H | 4'-$CO_2Me$ | H | H,H | H | H | Me |
| 51 | 6-F | 8-F | 4'-n-$C_3H_7$ | H | H,H | H | H | Me |
| 52 | 6-F | 8-F | 3'-I | 4'-OH | H,H | H | H | Me |
| 53 | 6-F | 8-F | 4'-I | H | H,H | H | H | Me |
| 54 | 6-$N_3$ | H | 4'-Cl | H | H,H | H | H | Me |
| 55 | 6-F | H | 4'-$N_3$ | H | H,H | H | H | Me |

**8.** Verwendung einer Verbindung der Formel (I) nach Anspruch 1 zur Herstellung eines Medikaments, das entweder zur Hemmung der Leukotriensynthese oder zur Antagonisierung von Prostaglandinen bei Säugern geeignet ist.

**9.** Pharmazeutisches Präparat, das ein Präparat nach Anspruch 1 und eine wirksame Menge eines zweiten aktiven Bestandteils enthält, der aus der Gruppe ausgewählt ist, bestehend aus nicht-steroiden, antiinflammatorischen Arzneimitteln, peripheren Analgetica, Cyclooxygenase-Inhibitoren, Leukotrien-Antagonisten, Leukotrien-Biosynthese-Hemmern, $H_1$-Rezeptor-Antagonisten, $H_2$-Rezeptor-Antagonisten, Prostaglandin-Antagonisten, ACE-Hemmern, Thromboxansynthetase-Inhibitoren oder Serotonin-Antagonisten.

**10.** Verwendung einer Verbindung nach Anspruch 2 zur Herstellung eines Medikamentes, das entweder zur Hemmung der Leukotriensynthese oder zur Antagonisierung von Prostaglandinen bei Säugern geeignet ist.

## Revendications

**1.** Composition pharmaceutique pour inhiber la synthèse des leucotriènes et antagoniser les prostaglandines chez les mammifères, comprenant une quantité efficace d'un composé de formule :

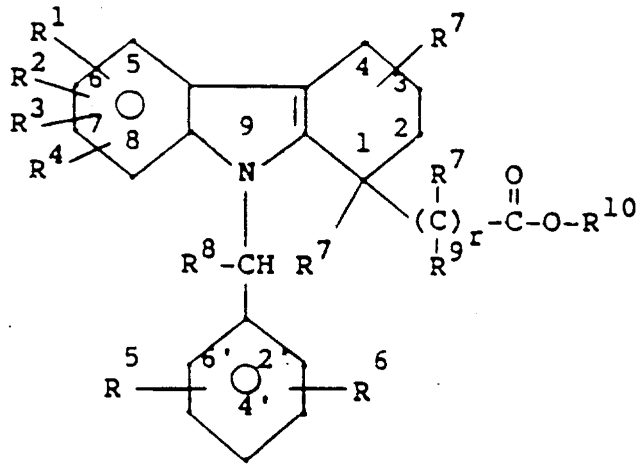

I

dans laquelle :

$R^1$, $R^2$, $R^3$, $R^4$, $R^5$ et $R^6$ sont chacun choisis indépendamment parmi :

(1) un atome d'hydrogène;

(2) un groupe alkyle comportant 1 à 6 atomes de carbone;

(3) un groupe alcényle comportant 2 à 6 atomes de carbone;

(4) $-(CH_2)_nM$

où n a une valeur de 0 à 3 et M est

a) $OR^{11}$;

b) un atome d'halogène;

c) $CF_3$;

d) $SR^{11}$;

e) un groupe phényle ou phényle substitué;

f) $COOR^{12}$;

g)

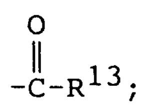

h) un groupe tétrazole;

i)

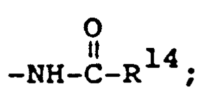

j) $-NR^{12}R^{12}$;

k) $-NHSO_2R^{15}$;

l)

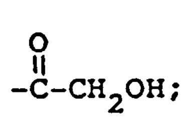

m) $-SOR^{11}$;

n) $-CONR^{12}R^{12}$;

o) $-SO_2NR^{12}R^{12}$;

p) $-SO_2R^{11}$;
q) $NO_2$;
r)

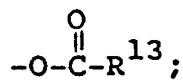

s)

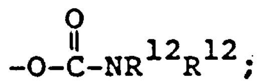

t)

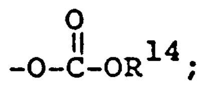

u) CN;
v) $N_3$;

$R^7$ est H ou un groupe alkyle de 1 à 6 atomes de carbone;

$R^8$ est H ou un groupe alkyle de 1 à 6 atomes de carbone;

chaque $R^9$ est indépendamment H, OH, un groupe -O-alkyle en $C_1$ à $C_4$ ou un groupe alkyle de 1 à 4 atomes de carbone;

$R^{10}$ est un groupe alkyle en $C_1$-$C_6$, un groupe 2-phénéthyle substitué ou non substitué, un groupe benzyle substitué ou non substitué, un groupe phényle substitué ou non substitué, ou

$-(CH_2)_t-C(R^7)_2-(CH_2)_t-R^{16}$

chaque $R^{11}$ est indépendamment H, ou un groupe alkyle en $C_1$ à $C_6$, benzyle, phényle ou phényle substitué;

chaque $R^{12}$ est indépendamment H, ou un groupe phényle, benzyle ou alkyle en $C_1$ à $C_6$;

chaque $R^{13}$ est indépendamment H, ou un groupe $(CH_2)_mCOOR^{12}$, dans lequel m a une valeur de 0 à 4, alkyle en $C_1$ à $C_6$, $CF_3$, phényle ou phényle substitué;

chaque $R^{14}$ est indépendamment un groupe alkyle en $C_1$ à $C_6$, benzyle ou phényle;

chaque $R^{15}$ est indépendamment un groupe alkyle en $C_1$ à $C_6$, 4-méthylphényle, phényle ou $CF_3$;

$R^{16}$ est A) un radical hétérocyclique, monocyclique ou bicyclique, contenant de 3 à 12 atomes de carbone dans le noyau et 1 ou 2 hétéroatomes dans le noyau, choisis parmi N, S ou O, chaque noyau du radical hétérocyclique étant formé de 5 ou 6 atomes, ou B) le radical $W-R^{17}$;

$R^{17}$ contient jusqu'à 21 atomes de carbone et est (1) un radical hydrocarboné ou (2) un radical acyle d'un acide carboxylique acyclique ou monocyclique organique ne contenant pas plus de 1 hétéroatome dans le noyau;

W est O, S ou NH;

r a une valeur de 1 à 6;

t a une valeur de 0 à 3;

les groupes phényle substitué, benzyle substitué et phénéthyle substitué indiqués ci-dessus étant substitués sur le noyau benzénique par 1 ou 2 substituants choisis parmi des groupes alkyle en $C_1$ à $C_3$, des atomes d'halogène, ou des groupes CN, $CF_3$, $COOR^{12}$, $CH_2COOR^{12}$ ou alcoxy en $C_1$ à $C_3$;
ou un de ses sels pharmaceutiquement acceptables;
et un véhicule pharmaceutiquement acceptable.

2. Composé de formule :

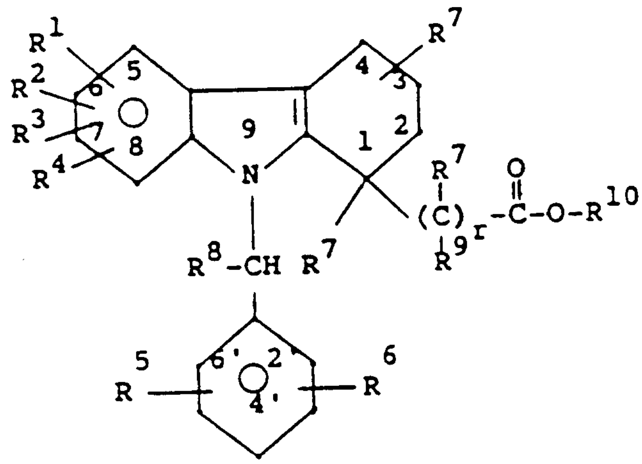

I

$R^1$, $R^2$, $R^3$, $R^4$, $R^5$ et $R^6$ sont chacun choisis indépendamment parmi :
(1) un atome d'hydrogène;
(2) un groupe alkyle comportant 1 à 6 atomes de carbone;
(3) un groupe alcényle comportant 2 à 6 atomes de carbone;
(4) $-(CH_2)_nM$
où n a une valeur de 0 à 3 et M est
a) $OR^{11}$;
b) un atome d'halogène;
c) $CF_3$;
d) $SR^{11}$;
e) un groupe phényle ou phényle substitué;
f) $COOR^{12}$;
g)

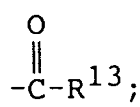

h) un groupe tétrazole;
i)

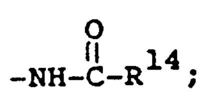

j) $-NR^{12}R^{12}$;
k) $-NHSO_2R^{15}$;
l)

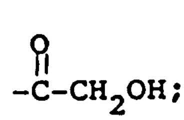

m) $-SOR^{11}$;
n) $-CONR^{12}R^{12}$;
o) $-SO_2NR^{12}R^{12}$;

p) $-SO_2R^{11}$;
q) $NO_2$;
r)

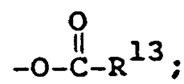

s)

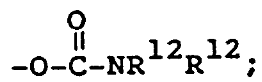

t)

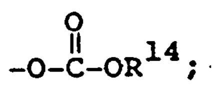

u) CN;
v) $N_3$;

à condition que, lorsque $R^1$, $R^2$, $R^3$ ou $R^4$ est un groupe alkyle comportant 1 à 6 atomes de carbone, il ne soit pas situé en position 6;

$R^7$ est H ou un groupe alkyle de 1 à 6 atomes de carbone;

$R^8$ est H ou un groupe alkyle de 1 à 6 atomes de carbone;

chaque $R^9$ est indépendamment H, OH, un groupe -O-alkyle en $C_1$ à $C_4$ ou un groupe alkyle de 1 à 4 atomes de carbone;

$R^{10}$ est un groupe alkyle en $C_1$-$C_6$, un groupe 2-phénéthyle substitué ou non substitué, un groupe benzyle substitué ou non substitué, un groupe phényle substitué ou non substitué, ou

$-(CH_2)_t-C(R^7)_2-(CH_2)_tR^{16}$

chaque $R^{11}$ est indépendamment H, ou un groupe alkyle en $C_1$ à $C_6$, benzyle, phényle ou phényle substitué;

chaque $R^{12}$ est indépendamment H, ou un groupe phényle, benzyle ou alkyle en $C_1$ à $C_6$; et

chaque $R^{13}$ est indépendamment H, ou un groupe $(CH_2)_mCOOR^{12}$, dans lequel m a une valeur de 0 à 4, alkyle en $C_1$ à $C_6$, $CF_3$, phényle ou phényle substitué;

chaque $R^{14}$ est un groupe alkyle en $C_1$ à $C_6$, benzyle ou phényle;

chaque $R^{15}$ est un groupe alkyle en $C_1$ à $C_6$, 4-méthylphényle, phényle ou $CF_3$;

$R^{16}$ est A) un radical hétérocyclique, monocyclique ou bicyclique, contenant de 3 à 12 atomes de carbone dans le noyau et 1 ou 2 hétéroatomes dans le noyau, choisis parmi M, S ou O, chaque noyau du radical hétérocyclique étant formé de 5 ou 6 atomes, ou B) le radical $W-R^{17}$;

$R^{17}$ contient jusqu'à 21 atomes de carbone et est (1) un radical hydrocarboné ou (2) un radical acyle d'un acide carboxylique acyclique ou monocyclique organique ne contenant pas plus de 1 hétéroatome dans le noyau;

W est O, S ou NH;

r a une valeur de 1 à 6;

t a une valeur de 0 à 3;

les groupes phényle substitué, benzyle substitué et phénéthyle substitué indiqués ci-dessus étant substitués sur le noyau benzénique par 1 ou 2 substituants choisis parmi des groupes alkyle en $C_1$ à $C_3$, des atomes d'halogène, ou des groupes CN, $CF_3$, $COOR^{12}$, $CH_2COOR^{12}$ ou alcoxy en $C_1$ à $C_3$; ou un de ses sels pharmaceutiquement acceptables.

**3.** Composé selon la revendication 2, qui est :

le 9-p-chlorobenzyl-6-fluoro-1,2,3,4-tétrahydrocarbazole-1-yl-acétate d'éthyle;

le 3-(9-p-chlorobenzyl-6-fluoro-1,2,3,4-tétrahydrocarbazole-1-yl)propanoate de méthyle;

le 3-(9-p-chlorobenzyl-6-méthoxy-1,2,3,4-tétrahydrocarbazole-1-yl)propanoate de méthyle;

le 9-p-chlorobenzyl-6-méthoxy-1,2,3,4-tétrahydrocarbazole-1-yl-acétate d'éthyle;
le 9-benzyl-6-fluoro-1,2,3,4-tétrahydrocarbazole-1-yl-acétated'éthyle;
le 9-p-méthoxybenzyl-6-fluoro-1,2,3,4-tétrahydrocarbazole-1-yl-acétate d'éthyle;
le 9-(3,4-dichloro)benzyl-6-fluoro-1,2,3,4-tétrahydrocarbazole-1-yl-acétate d'éthyle;
le 9-[1-(1-phényl)éthyl]-6-fluoro-1,2,3,4-tétrahydrocarbazole-1-yl-acétate d'éthyle;
le 9-p-chlorobenzyl-1,2,3,4-tétrahydrocarbazole-1-yl-acétate d'éthyle;
le 9-p-chlorobenzyl-6-chloro-1,2,3,4-tétrahydrocarbazole-1-yl-acétate d'éthyle;
le 9-p-chlorobenzyl-8-méthyl-1,2,3,4-tétrahydrocarbazole-1-yl-acétate d'éthyle;
le 6-bromo-9-p-chlorobenzyl-1,2,3,4-tétrahydrocarbazole-1-yl-acétate d'éthyle;
le 2-(9-p-chlorobenzyl-6-fluoro-1,2,3,4-tétrahydrocarbazole-1-yl)propanoate de méthyle;
le 9-p-chlorobenzyl-8-fluoro-1,2,3,4-tétrahydrocarbazole-1-yl-acétate d'éthyle;
le 9-p-chlorobenzyl-5,7-dichloro-1,2,3,4-tétrahydrocarbazole-1-yl-acétate d'éthyle;
le 9-p-chlorobenzyl-6,8-dichloro-1,2,3,4-tétrahydrocarbazole-1-yl-acétate d'éthyle;
le 9-p-chlorobenzyl-6-isopropyl-1,2,3,4-tétrahydrocarbazole-1-yl-acétate d'éthyle;
le 9-p-chlorobenzyl-6-t-butyl-1,2,3,4-tétrahydrocarbazole-1-yl-acétate d'éthyle;
le 9-p-chlorobenzyl-6-trifluorométhyl-1,2,3,4-tétrahydrocarbazole-1-yl-acétate d'éthyle;
le 9-p-chlorobenzyl-6-méthylthio-1,2,3,4-tétrahydrocarbazole-1-yl-acétate d'éthyle;
le 9-p-chlorobenzyl-6-méthylsulfinyl-1,2,3,4-tétrahydrocarbazole-1-yl-acétate d'éthyle;
le 9-p-chlorobenzyl-6-méthylsulfonyl-1,2,3,4-tétrahydrocarbazole-1-yl-acétate d'éthyle;
le 9-p-chlorobenzyl-8-isopropyl-1,2,3,4-tétrahydrocarbazole-1-yl-acétate d'éthyle;
l'ester méthylique d'acide 9-p-chlorobenzyl-8-méthylthio-1,2,3,4-tétrahydrocarbazole-1-yl-acétique;
l'ester méthylique d'acide 9-p-chlorobenzyl-8-méthylsulfinyl-1,2,3,4-tétrahydrocarbazole-1-yl-acétique;
le 9-p-chlorobenzyl-6-fluoro-3-méthyl-1,2,3,4-tétrahydrocarbazole-1-yl-acétate d'éthyle;
l'ester méthylique d'acide 9-p-chlorobenzyl-6,8-difluoro-1,2,3,4-tétrahydrocarbazole-1-yl-acétique;
l'ester méthylique d'acide 9-p-chlorobenzyl-6,8-diméthyl-1,2,3,4-tétrahydrocarbazole-1-yl-acétique;
l'ester méthylique d'acide 9-p-chlorobenzyl-6-méthoxy-8-méthyl-1,2,3,4-tétrahydrocarbazole-1-yl-acétique;
l'ester méthylique d'acide (-) 9-p-chlorobenzyl-6,8-difluoro-1,2,3,4-tétrahydrocarbazole-1-yl-acétique;
l'ester méthylique d'acide (+) 9-p-chlorobenzyl-6,8-difluoro-1,2,3,4-tétrahydrocarbazole-1-yl-acétique;
l'ester méthylique d'acide (-) 9-p-chlorobenzyl-8-méthyl-1,2,3,4-tétrahydrocarbazole-1-yl-acétique;
l'ester méthylique d'acide (+) 9-p-chlorobenzyl-8-méthyl-1,2,3,4-tétrahydrocarbazole-1-yl-acétique;
l'ester méthylique d'acide (-) 9-p-chlorobenzyl-8-fluoro-1,2,3,4-tétrahydrocarbazole-1-yl-acétique;
l'ester méthylique d'acide (+) 9-p-chlorobenzyl-8-fluoro-1,2,3,4-tétrahydrocarbazole-1-yl-acétique.

**4.** Composé selon la revendication 2, qui est :
le 9-p-chlorobenzyl-6-fluoro-1,2,3,4-tétrahydrocarbazole-1-yl-acétate d'éthyle;
le 9-p-chlorobenzyl-8-méthyl-1,2,3,4-tétrahydrocarbazole-1-yl-acétate d'éthyle;
le 9-p-chlorobenzyl-8-fluoro-1,2,3,4-tétrahydrocarbazole-1-yl-acétate d'éthyle;
l'ester méthylique d'acide 9-p-chlorobenzyl-6,8-difluoro-1,2,3,4-tétrahydrocarbazole-1-yl-acétique.

**5.** Composé selon la revendication 2, qui est :
l'ester méthylique d'acide (-) 9-p-chlorobenzyl-6-fluro-1,2,3,4,-tétrahydrocarbazole-1-yl-acétique;
l'ester méthylique d'acide (+) 9-p-chlorobenzyl-6-fluoro-1,2,3,4-tétrahydrocarbazole-1-yl-acétique;
l'ester méthylique d'acide (-) 9-p-chlorobenzyl-6-fluoro-1,2,3,4-tétrahydrocarbazole-1-yl-acétique;
l'ester méthylique d'acide (-) 9-p-chlorobenzyl-6,8-difluoro-1,2,3,4-tétrahydrocarbazole-1-yl-acétique;
l'ester méthylique d'acide (-) 9-p-chlorobenzyl-8-méthyl-1,2,3,4-tétrahydrocarbazole-1-yl-acétique;
l'ester méthylique d'acide (-) 9-p-chlorobenzyl-8-fluoro-1,2,3,4-tétrahydrocarbazole-1-yl-acétique.

**6.** Composé, qui est le 9-p-chlorobenzyl-6-méthyl-1,2,3,4,-tétrahydrocarbazole-1-yl-acétate d'éthyle.

**7.** Composé de formule :

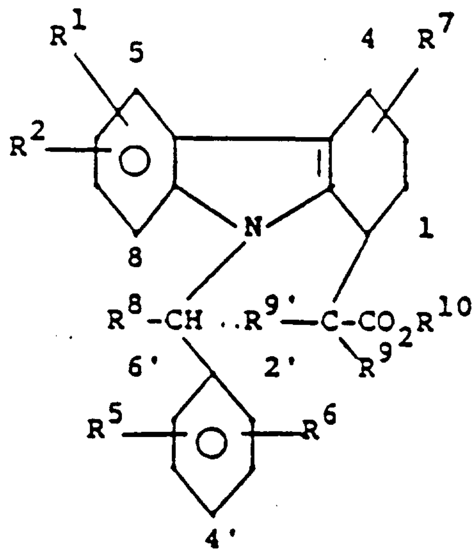

dans laquelle

| composé | $R^1$ | $R^2$ | $R^5$ | $R^6$ | $R^9$, $R^{9'}$ | $R^7$ | $R^8$ | $R^{10}$ |
|---|---|---|---|---|---|---|---|---|
| 1 (Ex.1) | 6-F | H | 4'-Cl | H | H, H | H | H | Et |
| 2 (Ex. 4) | 6-OMe | H | 4'-Cl | H | H, H | H | H | Et |
| 3 (Ex. 18) | 6-F (-) isomère | H | 4'-Cl | H | H, H | H | H | Me |
| 4 (Ex. 19) | 6-F (+) isomère | H | 4'-Cl | H | H, H | H | H | Me |
| 5 (Ex. 5) | 6-F | H | H | H | H, H | H | H | Et |
| 6 (Ex. 6) | 6-F | H | 4'-OMe | H | H, H | H | H | Et |
| 7 (Ex. 7) | 6-F | H | 3'-Cl | 4'-Cl | H, H | H | H | Et |
| 8 (Ex. 8) | 6-F | H | H | H | H, H | H | Me | Et |
| 9 (Ex 9) | H | H | 4'-Cl | H | H, H | H | H | Et |
| 10 (Ex. 10) | 6-Cl | H | 4'-Cl | H | H, H | H | H | Et |
| 11 (Ex. 11) | 8-Me | H | 4'-Cl | H | H, H | H | H | Et |
| 12 (Ex. 12) | 6-Br | H | 4'-Cl | H | H, H | H | H | Et |

| composé | $R^1$ | $R^2$ | $R^5$ | $R^6$ | $R^9$, $R^{9'}$ | $R^7$ | $R^8$ | $R^{10}$ |
|---|---|---|---|---|---|---|---|---|
| 13 (Ex. 14) | 8-F | H | 4'-Cl | H | H, H | H | H | Et |
| 14 | 6-F | H | 4'-Cl | H | H, H | 3-t-Bu | H | $CH_2C_6H_5$ |
| 15 | 5-F | H | 4'-Cl | H | H, H | H | H | $CH_2OAc$ |
| 16 | 7-F | H | 4'-Cl | H | H, H | H | H | 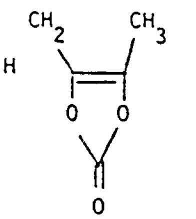 |
| 17 (Ex. 15) | 5-Cl | 7-Cl | 4'-Cl | H | H, H | H | H | Et |
| 18 (Ex. 16) | 6-Cl | 8-Cl | 4'-Cl | H | H, H | H | H | Et |
| 19 | 6-F | H | 4'-Cl | H | Me, H | H | H | 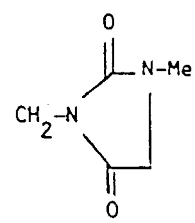 |
| 20 | 6-F | H | 4'-Cl | H | Me, Me | H | H | 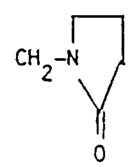 |
| 21 | 6-F | H | 4'-Cl | H | H, H | 1-Me | H | 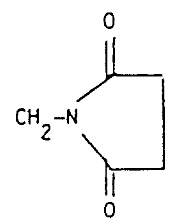 |

| composé | $R^1$ | $R^2$ | $R^5$ | $R^6$ | $R^9,R^{9'}$ | $R^7$ | $R^8$ | $R^{10}$ |
|---|---|---|---|---|---|---|---|---|
| 22 (ex 19) | 6-$CF_3$ | H | 4'-Cl | H | H,H | H | H | Et |
| 23 (ex 20) | 6-SMe | H | 4'-Cl | H | H,H | H | H | Et |
| 24 (ex 21) | 6-SOMe | H | 4'-Cl | H | H,H | H | H | Et |
| 25 (ex 22) | 6-$SO_2$Me | H | 4'-Cl | H | H,H | H | H | Et |
| 26 (ex 23) | 8-CH$(Me)_2$ | H | 4'-Cl | H | H,H | H | H | Et |
| 27 (ex 24) | 8-SMe | H | 4'-Cl | H | H,H | H | H | Me |
| 28 (ex 25) | 8-SOMe | H | 4'-Cl | H | H,H | H | H | Me |
| 29 (ex 26) | 6-F | H | 4'-Cl | H | H,H | 3-Me | H | Et |
| 30 (ex 27) | 6-F | 8-F | 4'-Cl | H | H,H | H | H | Me |
| 31 (ex 28) | 6-OMe | 8-Me | 4'-Cl | H | H,H | H | H | Me |
| 32 (ex 29) | 6-F(-)Isomère | 8-F | 4'-Cl | H | H,H | H | H | Me |
| 33 (ex 30) | 6-F(+)Isomère | 8-F | 4'-Cl | H | H,H | H | H | Me |
| 34 (ex 31) | 8-Me(-)Isomère | H | 4'-Cl | H | H,H | H | H | Me |
| 35 (ex 32) | 8-Me(+)Isomère | H | 4'-Cl | H | H,H | H | H | Me |
| 36 (ex 33) | 8-F(-)Isomère | H | 4'-Cl | H | H,H | H | H | Me |
| 37 (ex 34) | 8-F(+)Isomère | H | 4'-Cl | H | H,H | H | H | Me |
| 38 | 6-F | 8-F | 3'-Cl | 4'-Cl | H,H | H | H | Me |
| 39 | 6-F | 8-F | 2'-Cl | 4'-Cl | H,H | H | H | Me |
| 40 | 6-F | 8-F | 4'-OMe | H | H,H | H | H | Me |
| 41 | 6-F | 8-F | 4'-OH | H | H,H | H | H | Me |
| 42 | 6-F | 8-F | 4'-SMe | H | H,H | H | H | Me |
| 43 | 6-F | H | 4'-S(O)Me | H | H,H | H | H | Me |
| 44 | 6-F | 8-F | 4'-NHCOMe | H | H,H | H | H | Me |
| 45 | 6-F | H | 4'-$S(O)_2$Me | H | H,H | H | H | Me |

| composé | $R^1$ | $R^2$ | $R^5$ | $R^6$ | $R^9,R^{9'}$ | $R^7$ | $R^8$ | $R^{10}$ |
|---|---|---|---|---|---|---|---|---|
| 46 | 6-F | H | 4'-F | H | H,H | H | H | Me |
| 47 | 6-F | H | 4'-Br | H | H,H | H | H | Me |
| 48 | 6-F | 8-Me | 4'-Cl | H | H,H | H | H | Me |
| 49 | 6-F | H | 4'-$CO_2H$ | H | H,H | H | H | Me |
| 50 | 6-F | H | 4'-$CO_2Me$ | H | H,H | H | H | Me |
| 51 | 6-F | 8-F | 4'-n-$C_3H_7$ | H | H,H | H | H | Me |
| 52 | 6-F | 8-F | 3'-I | 4'-OH | H,H | H | H | Me |
| 53 | 6-F | 8-F | 4'-I | H | H,H | H | H | Me |
| 54 | 6-$N_3$ | H | 4'-Cl | H | H,H | H | H | Me |
| 55 | 6-F | H | 4'-$N_3$ | H | H,H | H | H | Me |

8. Utilisation d'un composé de formule (I) selon la revendication 1, pour la fabrication d'un médicament utile pour inhiber la synthèse des leucotriènes ou pour antagoniser les prostaglandines chez les mammifères.

9. Composition pharmaceutique, qui comprend une composition selon la revendication 1 et une quantité efficace d'un second ingrédient actif choisi dans le groupe constitué par les anti-inflammatoires non stéroïdiens, les analgésiques périphériques, les inhibiteurs de cyclooxygénase, les antagonistes de leucotriène, les inhibiteurs de la biosynthèse des leucotriènes, les antagonistes des récepteurs $H_1$, les antagonistes des récepteurs $H_2$, les antagonistes de prostaglandine, les inhibiteurs d'ACE, les inhibiteurs de thromboxane synthétase et les antagonistes de sérotonine.

10. Utilisation d'un composé selon la revendication 2 pour la fabrication d'un médicament utile pour inhiber la synthèse des leucotriènes ou pour antagoniser les prostaglandines chez les mammifères.